# EUROPÄISCHE PATENTSCHRIFT

(11) **EP 3 826 986 B1**
(45) Veröffentlichungstag und Bekanntmachung des Hinweises auf die Patenterteilung: **04.03.2026**
(21) Anmeldenummer: 18745901.1
(22) Anmeldetag: 23.07.2018
(51) Int. Cl.: C07C 49/403, C07C 47/32, C07C 31/135, C07C 35/08, C11B 9/00, C07C 35/17, C07C 35/18

(54) **NEUE RIECHSTOFFE MIT ROSENGERUCH**
NOVEL ODORANTS HAVING ROSE ODOUR
NOUVELLES SUBSTANCES ODORANTES PRÉSENTANT UNE ODEUR DE ROSE

(43) Veröffentlichungstag der Anmeldung: 02.06.2021
(73) Patentinhaber: Symrise AG, 37603 Holzminden (DE)
(72) Erfinder: HÖLSCHER, Bernd, 37620 Halle (DE); CHANDRASEKARAN, Vijayanand, 37603 Holzminden (DE)
(74) Vertreter: Daniels, Stefanie Lisa
(86) Internationale Anmeldenummer: PCT/EP2018/069950
(87) Internationale Veröffentlichungsnummer: WO 2020/020434

(56) Entgegenhaltungen:
- WO-A1-2007/009982
- DE-A1- 2 744 134
- KAKU H ET AL: "A facile and practical method of preparing optically active @a-monosubstituted cycloalkanones by thermodynamically controlled deracemization", TETRAHEDRON, ELSEVIER SCIENCE PUBLISHERS, AMSTERDAM, NL, vol. 66, no. 48, 27 November 2010 (2010-11-27), pages 9450 - 9455, XP027452245, ISSN: 0040-4020, [retrieved on 20101001], DOI: 10.1016/J.TET.2010.09.085
- FERINGA ET AL.: "Copper-catalyzed asymmetric conjugate addition of Grignard reagents to cyclic enones", PROCEEDINGS OF RGE NATIONAL ACADEMY OF SCIENCES, vol. 101, no. 16, 20 April 2004 (2004-04-20), pages 5834 - 5838, XP002790192
- HARTUNG ET AL.: "(Schiff-base)vanadium(V) Complex-Catalyzed Oxidations of Susbstituted Bis(homoallylic) Alcohols - Stereoselective Synthesis of Functionalized Tetrahydrofurans", EUR. J. ORG. CHEM., vol. 2003, no. 23, 1 July 2003 (2003-07-01), pages 2388 - 2408, XP002790193
- CHARLES DESCOINS ET AL: "No 310. - Transposition homoallylique avec participation. II. - lsomerisation acide des [(pentene-4" yl)-2'].cyclopropyl.2 propanols-2 (b) (cis et trans) (*) (**) - [Homoallylic rearrangement with participating groups]", BULLETIN DE LA SOCIETE CHIMIQUE DE FRANCE, SOCIETE FRANCAISE DE CHIMIE. PARIS, FRANCE, no. 5, 1 January 1970 (1970-01-01), pages 1816 - 1822, XP009509756, ISSN: 0037-8968

## Beschreibung

Die vorliegende Erfindung betrifft primär die Verwendung von Verbindungen der nachfolgenden Formel (I) als Riech- und/oder Aromastoffe. Die Erfindung betrifft zudem Riech- und/oder Aromastoffkompositionen enthaltend ein oder mehrere dieser Verbindungen, parfümierte und aromatisierte Artikel umfassend ein oder mehrere dieser Verbindungen sowie entsprechende Verfahren zum Vermitteln, Modifizieren und/oder Verstärken bestimmter Geruchs- und/oder Geschmacksnoten.

In US 2010/0130397 wird beispielsweise die Verwendung von 4-Isoamylcyclohexanol als Aromastoff beschrieben. Die WO 2007/009982 A1 offenbart die Verwendung von 4-Isoamylcyclohexanon als Riech- oder Aromastoff mit Rosen-Geranium-Note.

Trotz der Vielzahl bereits vorhandener Riech- und Aromastoffe besteht in der Parfüm- und Aromen-Industrie auch weiterhin ein genereller Bedarf an neuen Riech- und Aromastoffen. So besteht beispielsweise ein Bedarf an Riech- oder Aromastoffen, die in der Lage sind (in Riech- oder Aromastoffkompositionen) neben einer primären Duft- oder Geschmacksnote weitere interessante Noten zu erzeugen und mit ihren neuartigen bzw. originellen olfaktorischen Eigenschaften die Möglichkeiten des Parfümeurs zu erweitern. Insbesondere besteht ein Interesse an Riech und/oder Aromastoffen mit Duftnoten, welche in der Lage sind, eine harmonische Kombination mit blumigen und/oder fruchtigen Riech- und/oder Aromastoffen einzugehen. Vorzugsweise sollte dabei eine Überlagerung der unterschiedlichen geruchlichen Aspekte und Noten erfolgen, um dadurch einen insgesamt komplexen Geruchseindruck zu erzeugen.

Für die Kreation neuartiger Kompositionen besteht ständiger Bedarf an Riech- und/oder Aromastoffen mit besonderen sensorischen Eigenschaften, die geeignet sind, als Grundlage für die Komposition von neuartigen Parfüms mit komplexem sensorischen Charakter zu dienen. Bevorzugte gesuchte Riech- und/oder Aromastoffe sollten neben einer bestimmten Note weitere Noten und Aspekte aufweisen, die ihnen Charakter und Komplexität verleihen.

Die Suche nach geeigneten rosenartigen Stoffen, die zur vorliegenden Erfindung führte, wurde durch folgende Sachverhalte erschwert:
i) die Mechanismen der Geruchswahrnehmung sind nicht ausreichend bekannt;
ii) die Zusammenhänge zwischen der speziellen Geruchswahrnehmung einerseits und der chemischen Struktur des zugehörigen Riech- und/oder Aromastoffs andererseits sind nicht hinreichend erforscht;
iii) häufig bewirken bereits geringfügige Änderungen am strukturellen Aufbau eines bekannten Riech- und/oder Aromastoffs starke Änderungen der sensorischen Eigenschaften und beeinträchtigen die Verträglichkeit für den menschlichen Organismus.

Der Erfolg der Suche nach geeigneten Riech- und/oder Aromastoffen hängt deshalb stark von der Intuition des Suchenden ab.

Die primäre Aufgabe bestand nun darin, Riech- und/oder Aromastoffe zu finden, die ein interessantes, vorzugsweise komplexes, und originelles sensorisches Profil aufweisen und sich als Riech- oder Aromastoffe für den Einsatz in der Parfümerie eignen.

Es wurden im Rahmen der vorliegenden Erfindung insbesondere Stoffe gesucht, die eine, mehrere oder sämtliche der der Noten grün, krautig, frisch, fruchtig, blumig, holzig, süß, erdig, fettig, metallisch und balsamisch aufweisen bzw. vermitteln, modifizieren und/oder verstärken können. Insbesondere sollten Stoffe mit Noten gefunden werden, welche vorzugsweise zumindest einer der Noten blumig und/oder fruchtig aufweisen und bevorzugt zudem rosenartig riechen bzw. eine Rosen-Note vermitteln können.

Die gesuchten Stoffe sollten die Herstellung von neuartigen Riech- und/oder Aromastoffkompositionen mit besonderen geruchlichen Noten und Aspekten ermöglichen. Von Vorteil wären dabei solche Stoffe, welche insbesondere zur Kombination mit weiteren Riech- und/oder Aromastoffen geeignet sind, die eine holzige und/oder blumige und/oder fruchtige Note aufweisen.

Daneben sollten die diese primäre Aufgabe erfüllenden Riech- und/oder Aromastoffe vorzugsweise über ihre primären, nämlich geruchlichen, Eigenschaften hinaus zusätzliche positive Sekundäreigenschaften besitzen, wie z.B. eine hohe Stabilität unter bestimmten Anwendungsbedingungen, eine hohe Ausgiebigkeit, ein gutes Haftungsvermögen, eine hohe Substantivität, geruchsverstärkende Eigenschaften aufweisen (sogenannte Booster- oder Enhancer-Wirkung) und/oder in Kombination mit anderen Riech- und/oder Aromastoffen deren Natürlichkeit, Frische, Fülle, (Strahl)Kraft und/oder Ausstrahlung abzurunden, so dass sensorisch bemerkenswerte Effekte erzielt werden können.

Eine weitere Aufgabe der Erfindung bezieht sich auf die Herstellung solcher neuartigen Riech- und/oder Aromastoffe in jeglicher Form, ihrer Isomere, sowie deren Mischungen.

Eine weitere Aufgabe betrifft die Bereitstellung von neuen Verbindungen zur Verwendung als Riech- und Aromastoffe, von bevorzugten Mischungen, sowie von diesen Mischungen enthaltenden Konsumgüter und deren Herstellung.

Diese Aufgaben werden durch die in den Hauptansprüchen definierten Merkmalskombinationen gelöst. Bevorzugte Ausführungsformen sind Gegenstand der Unteransprüche.

### Zusammenfassung der Erfindung

Die Erfindung betrifft in einem ersten Aspekt die Verwendung einer Verbindung der Formel (I), 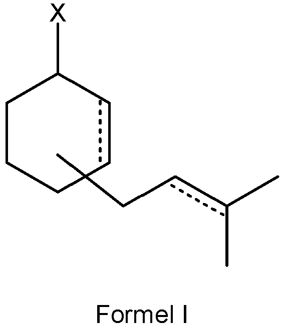 wobei X eine -CHO, eine -OH, oder eine -CH₂OH Gruppe darstellt, am jeweiligen Ort einer der gestrichelt dargestellten Linien jeweils eine Einfachbindung oder eine Doppelbindung vorliegt, wobei wenn X eine OH-Gruppe ist, mindestens eine Doppelbindung vorliegt, und wobei der 3-Isopentyl- oder 3-Isopent-2-enyl-Rest in ortho-, meta-, oder para-Stellung zur X-Gruppe mit dem Ring verbunden ist unter der Voraussetzung, dass wenn eine Doppelbindung im Ring vorhanden ist, dann der 3-Isopentyl- oder 3-Isopent-2-enyl-Rest als ortho- oder meta-Substituent direkt an dieser Doppelbindung gebunden ist, als Riech- und/oder Aromastoff.

Eine besonders bevorzugte Alternative ist hier und im Folgenden, die Verwendung einer Verbindung der Formel (la), 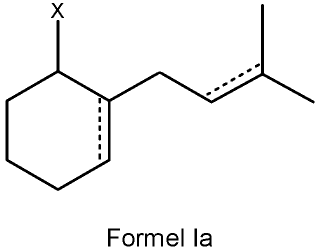 wobei X eine -CHO, eine -OH, oder eine -CH₂OH Gruppe darstellt, am jeweiligen Ort einer der gestrichelt dargestellten Linien jeweils eine Einfachbindung oder eine Doppelbindung vorliegt, wobei wenn X eine OH-Gruppe ist, mindestens eine Doppelbindung vorliegt, als Riech- und/oder Aromastoff.

Eine weitere besonders bevorzugte Alternative ist hier und im Folgenden, die Verwendung einer Verbindung der Formel (Ib), 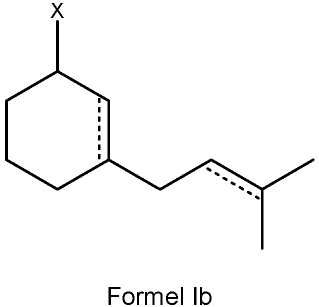 wobei X eine -CHO, eine -OH, oder eine -CH₂OH Gruppe darstellt, am jeweiligen Ort einer der gestrichelt dargestellten Linien jeweils eine Einfachbindung oder eine Doppelbindung vorliegt, wobei wenn X eine OH-Gruppe ist, mindestens eine Doppelbindung vorliegt, als Riech- und/oder Aromastoff.

Eine alternative besonders bevorzugte Alternative ist hier und im Folgenden, die Verwendung einer Verbindung der Formel (Ic), 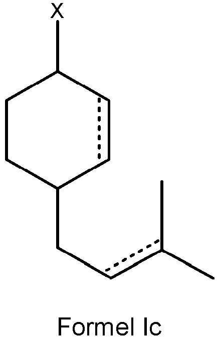 wobei X eine -CHO, eine -OH, oder eine -CH₂OH Gruppe darstellt, am jeweiligen Ort einer der gestrichelt dargestellten Linien jeweils eine Einfachbindung oder eine Doppelbindung vorliegt, wobei wenn X eine OH-Gruppe ist, mindestens eine Doppelbindung vorliegt, als Riech- und/oder Aromastoff.

Eine besonders bevorzugte Alternative der Formel (I), der Formel (la), der Formel (Ib), und/oder der Formel (Ic) ist hier und im Folgenden, dass die Doppelbindung eine "C=C" Doppelbindung ist.

Bevorzugt sind weiterhin die Verwendung der folgenden Molekülvarianten als Riech- und/oder Aromastoff:

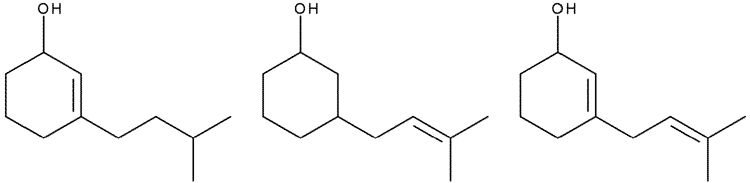

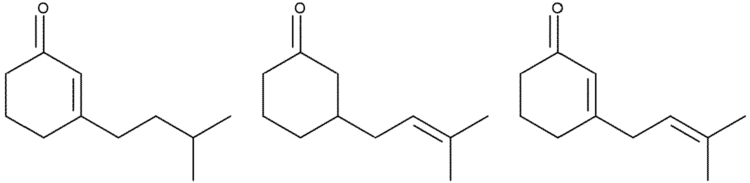

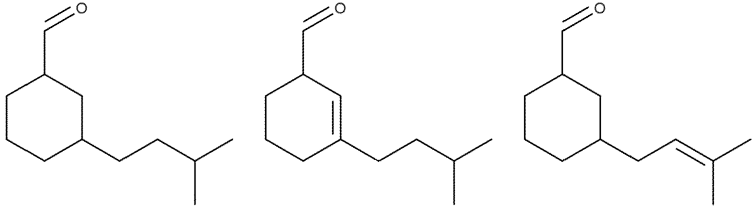

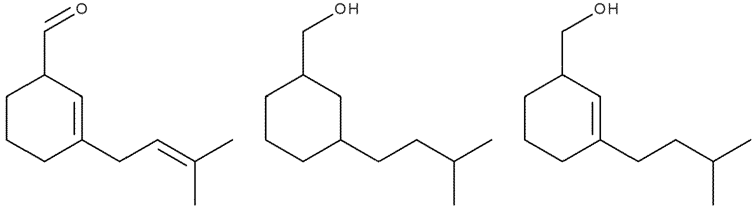

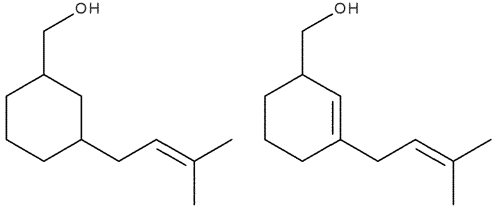

Die Molekülvarianten sind jeweils mit dem meta-Substituenten dargestellt, der Substituent kann aber auch jeweils bevorzugt in der ortho- oder para-Position gebunden sein, wie beispielsweise bei: 4-Isopentylcyclohex-2-en-1-ol, 2-Isopentylcyclohex-2-en-1-ol, 4-Isopentylcyclohex-2-en-1-on, oder 2-Isopentylcyclo-hex-2-en-1-on. Sämtliche oben gezeigten Beispielformeln sind daher auch mit ortho-oder para-Substituent bevorzugt.

Die Verbindungen der Formel (I), der Formel (la), der Formel (Ib), und/oder der Formel (Ic) verfügen über ganz eigenständige olfaktorische Eigenschaften, die sich deutlich von den bekannten Riech- und/oder Aromastoffen abheben und diese auch übertreffen. Die Eignung der Verbindungen der Formel (I), der Formel (la), der Formel (Ib), und/oder der Formel (Ic) als Riech- und/oder Aromastoffen war bislang nicht bekannt. Es ist daher besonders überraschend, dass diese wertvollen Riech- und/oder Aromastoffe mit interessanten und komplexen Geruchseigenschaften gefunden werden konnten.

Besonders bevorzugt ist die Verwendung der erfindungsgemäßen Verbindungen als Rosen-Riech- und/oder Aromastoffe.

Eine bevorzugte Variante der vorliegenden Erfindung betrifft die Verwendung einer Verbindung der Formel (I), der Formel (la), der Formel (Ib), und/oder der Formel (Ic), wobei X eine -CHO, eine -OH, oder eine -CH₂OH Gruppe darstellt, und am jeweiligen Ort einer der gestrichelt dargestellten Linien mindestens eine Doppelbindung vorliegt, als Riech- und/oder Aromastoff.

Besonders bevorzugt ist hierbei die Verwendung der folgenden Molekülvarianten als Riech- und/oder Aromastoff:

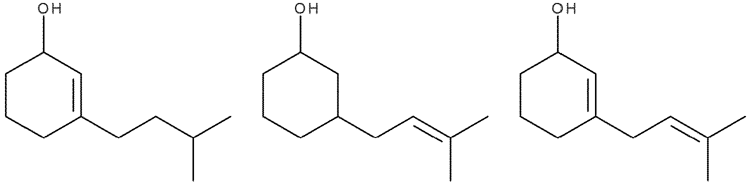

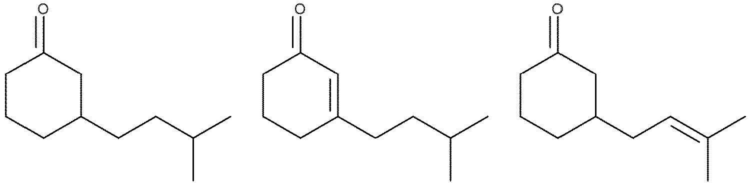

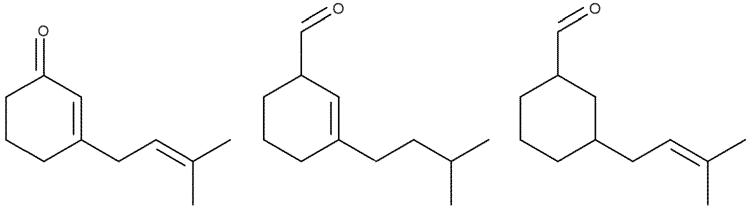

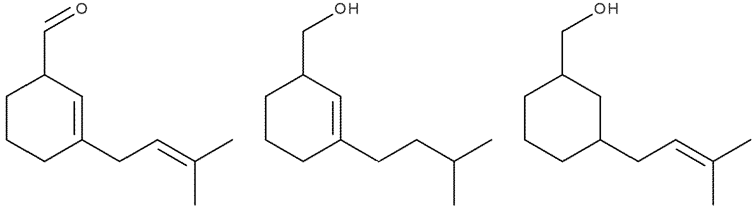

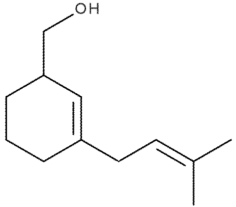

Die Molekülvarianten sind jeweils besonders bevorzugt mit dem meta-Substituenten dargestellt, der Substituent kann auch jeweils bevorzugt in der ortho- oder para-Position gebunden sein, wie beispielsweise bei: 4-Isopentylcyclohex-2-en-1-ol, 2-Isopentylcyclohex-2-en-1-ol, 4-Isopentylcyclohex-2-en-1-on, oder 2-Isopentylcyclo-hex-2-en-1-on. Sämtliche oben gezeigten Beispielformeln sind daher auch mit ortho-oder para-Substituent bevorzugt.

Diese Verbindungen sind besonders bevorzugt, da sie ein verstärktes Geruchsprofile nach rosig und fruchtig aufweisen. Zusätzlich besitzen sie positive Sekundäreigenschaften, wie z.B. eine hohe Stabilität unter bestimmten Anwendungsbedingungen.

Eine alternative bevorzugte Variante der vorliegenden Erfindung betrifft Verbindungen der Formel (I), der Formel (la), der Formel (Ib), und/oder der Formel (Ic), wobei X eine -CHO, eine -OH, oder eine -CH₂OH Gruppe darstellt, am jeweiligen Ort einer der gestrichelt dargestellten Linien im Ring und in der Isobutyl-Seitenkette jeweils eine Doppelbindung vorliegt, als Riech- und/oder Aromastoff.

Eine weitere bevorzugte Ausgestaltung der vorliegenden Erfindung betrifft Verbindungen der Formel (I), der Formel (la), der Formel (Ib), und/oder der Formel (Ic), wobei X eine -CHO, eine -OH, oder eine -CH₂OH Gruppe darstellt, am Ort einer der gestrichelt dargestellten Linien jeweils eine Einfachbindung oder eine Doppelbindung vorliegt, und wobei die Verbindung nicht 2-(3-Methylbutyl)cyclohexan-1-ol, 2-(3-Methylbutyl)cyclohexan-1-on 3-(3-Methylbutyl)cyclohexan-1-ol, 3-(3-Methylbutyl)cyclohexan-1-on 4-(3-Methylbutyl)cyclohexan-1-ol, oder 4-(3-Methylbutyl)cyclohexan-1-on ist, als Riech- und/oder Aromastoff.

Besonders bevorzugt ist hierbei die Verwendung der folgenden Molekülvarianten als Riech- und/oder Aromastoff:

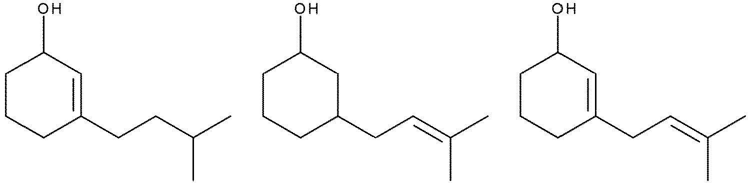

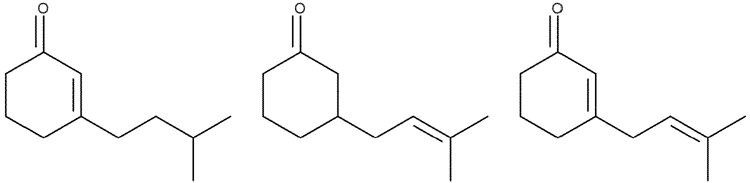

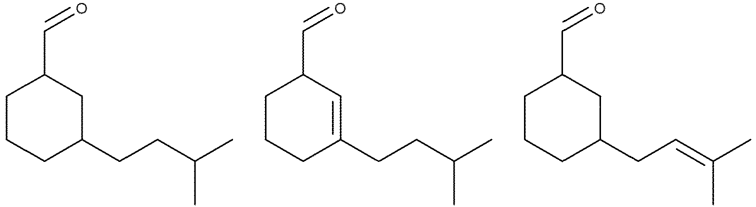

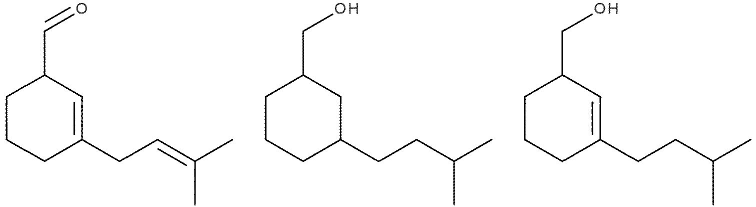

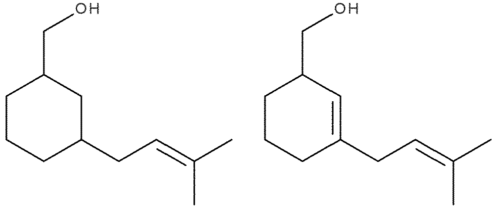

Die Molekülvarianten sind jeweils bevorzugt mit dem meta-Substituenten dargestellt, der Substituent kann auch jeweils in der ortho- oder para-Position gebunden sein, wie beispielsweise bei: 4-Isopentylcyclohex-2-en-1-ol, 2-Isopentylcyclohex-2-en-1-ol, 4-Isopentylcyclohex-2-en-1-on, oder 2-Isopentylcyclohex-2-en-1-on. Sämtliche oben gezeigten Beispielformeln sind daher auch mit ortho- oder para-Substituent bevorzugt.

Eine alternative bevorzugte Ausgestaltung der vorliegenden Erfindung betrifft Verbindungen der Formel (I), der Formel (la), der Formel (Ib), und/oder der Formel (Ic), wobei X eine -CHO, eine -OH, oder eine -CH₂OH Gruppe darstellt, am jeweiligen Ort einer der gestrichelt dargestellten Linien entweder im Ring oder in der Isobutyl-Seitenkette eine Doppelbindung vorliegt, als Riech- und/oder Aromastoff.

Diese Verbindungen sind besonders bevorzugt, da sie die Noten fruchtig, blumig und holzig besonders gut modifizieren und/oder verstärken können.

Die Erfindung betrifft in einem zweiten Aspekt, Verbindungen der Formel (II), 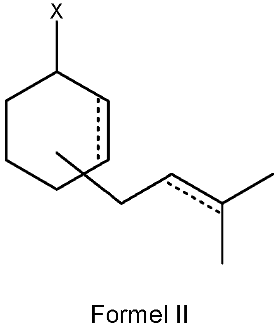 wobei, X eine -CHO oder eine -CH₂OH -Gruppe darstellt, am Ort einer der gestrichelt dargestellten Linien jeweils eine Einfachbindung oder eine Doppelbindung vorliegt und wobei der 3-Isopentyl- oder 3-Isopent-2-enyl-Rest in ortho-, meta-, oder para-Stellung zur X-Gruppe mit dem Ring verbunden ist unter der Voraussetzung, dass wenn eine Doppelbindung im Ring vorhanden ist, dann der 3-Isopentyl- oder 3-Isopent-2-enyl-Rest als ortho- oder meta-Substituent direkt an dieser Doppelbindung gebunden ist.

Die bevorzugten Ausgestaltungen und Strukturvarianten des ersten Aspekts der vorliegenden Erfindung treffen jeweils auch auf den zweiten Aspekt zu und sind auch mit der Grundstruktur nach Formel II sinngemäß kombinierbar.

Eine weitere bevorzugte Ausgestaltung der vorliegenden Erfindung betrifft in diesem Zusammenhang die folgenden Molekülvarianten:

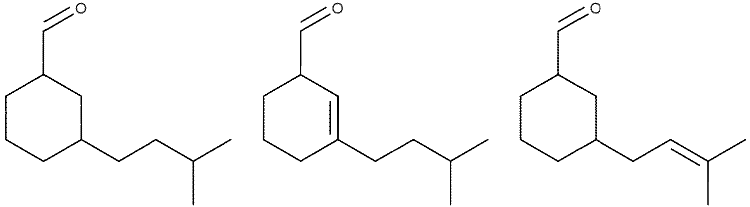

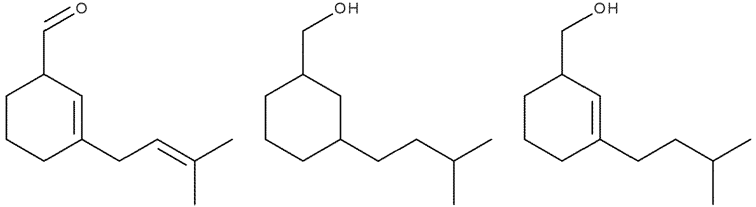

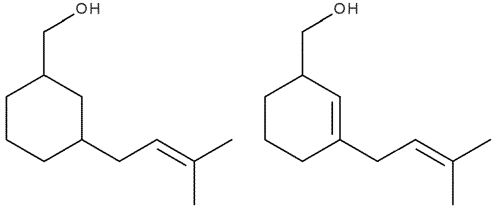

Die Verbindungen der Formel (II) weisen eine zusätzliches "Kohlenstoff"-Atom zwischen dem Ring und dem Heteroatom auf, wodurch sie sich besonders gut als Riechstoffe eignen. Darüber hinaus überzeugen die erfindungsgemäßen Verbindungen durch ein außergewöhnlich gutes Haftungsvermögen (long lasting) und eine hohe Substantivität.

Eine alternative bevorzugte Ausgestaltung der vorliegenden Erfindung betrifft Verbindungen der Formel (II), wobei X eine -CHO oder eine -CH₂OH Gruppe darstellt, am Ort der gestrichelt dargestellten Linien jeweils eine Einfachbindung vorliegt und der 3-Isopentyl- oder 3-Isopent-2-enyl-Rest in ortho-, meta-, oder para-Stellung zur X-Gruppe mit dem Ring verbunden ist unter der Voraussetzung, dass wenn eine Doppelbindung im Ring vorhanden ist, dann der 3-Isopentyl- oder 3-Isopent-2-enyl-Rest als ortho- oder meta-Substituent direkt an dieser Doppelbindung gebunden ist.

Eine bevorzugte Ausgestaltung der vorliegenden Erfindung betrifft die Verwendung einer Verbindung der Formel (II), 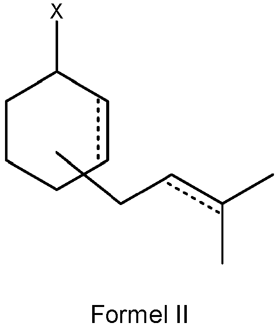 wobei X eine -CHO oder eine -CH₂OH Gruppe darstellt, am Ort der gestrichelt dargestellten Linien jeweils eine Einfachbindung oder eine Doppelbindung vorliegt und der 3-Isopentyl- oder 3-Isopent-2-enyl-Rest in ortho-, meta-, oder para-Stellung zur X-Gruppe mit dem Ring verbunden ist unter der Voraussetzung, dass wenn eine Doppelbindung im Ring vorhanden ist, dann der 3-Isopentyl- oder 3-Isopent-2-enyl-Rest als ortho- oder meta-Substituent direkt an dieser Doppelbindung gebunden ist, als Riech- und/oder Aromastoff.

Besonders bevorzugt ist hierbei die Verwendung der folgenden Molekülvarianten als Riech- und/oder Aromastoff:

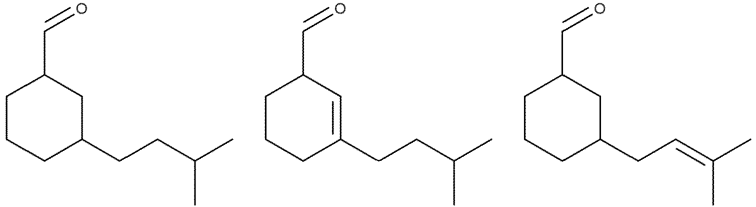

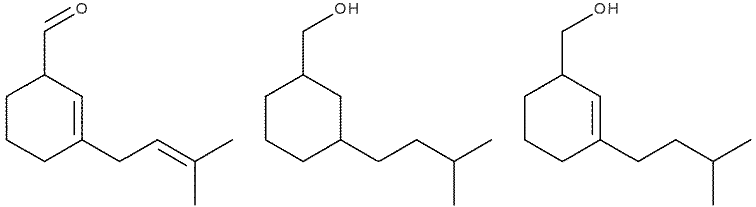

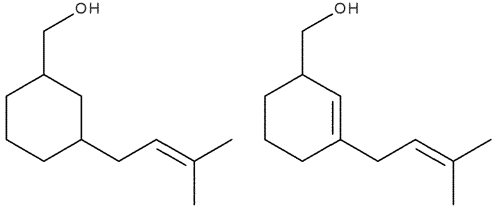

Diese Molekülvarianten sind jeweils bevorzugt mit dem meta-Substituenten dargestellt, der Substituent kann auch jeweils in der ortho- oder para-Position gebunden sein, wie beispielsweise bei: 2-Isopentylcyclohex-2-ene-1-carbaldehyde, 4-Isopentylcyclohex-2-ene-1-carbaldehyde, 2-(3-Methylbut-2-enyl)cyclohexancarbalde-hyde, 4-(3-Methylbut-2-enyl)cyclohexancarbaldehyde, [2-(3-Methylbut-2-enyl)cyclo-hexyl]methanol, [4-(3-Methylbut-2-enyl)cyclohexyl]methanol, (2-Isopentylcyclohex-2-en-1-yl)methanol oder (4-isopentylcyclohex-2-en-1-yl)methanol. Sämtliche oben gezeigten Beispielformeln sind daher auch mit ortho- oder para-Substituent bevorzugt.

Die Verbindungen der Formel (II) besitzen ein herausragendes und einzigartiges Geruchsprofil, welches u.a. rosige und blumige Noten aufweist. Darüber hinaus überzeugen die erfindungsgemäßen Verbindungen durch ein außergewöhnlich gutes Haftungsvermögen (long lasting) und eine hohe Substantivität.

Eine alternative bevorzugte Ausgestaltung der vorliegenden Erfindung betrifft Verbindungen der Formel (II), wobei X eine -CHO oder eine -CH₂OH Gruppe darstellt, am Ort der gestrichelt dargestellten Linien jeweils eine Einfachbindung vorliegt und der 3-Isopentyl- bzw. 3-Isopentenyl-Rest in ortho-, meta-, oder para-Stellung zur X-Gruppe am Ring verbunden ist, als Riech- und/oder Aromastoff.

Besonders bevorzugt ist hierbei die Verwendung der folgenden Molekülvarianten als Riech- und/oder Aromastoff: 2-Isopentylcyclohexancarbaldehyde, (2-Isopentylcyclohexyl)methanol, 3-Isopentylcyclohexancarbaldehyde, (3-Isopentylcyclohexyl)methanol, 4-Isopentylcyclohexancarbaldehyde und (4-Isopentyl-cyclohexyl)methanol.

Diese Verbindungen sind besonders bevorzugt, da sie geruchsverstärkende Eigenschaften aufweisen (sogenannte Booster- oder Enhancer-Wirkung) und/oder in Kombination mit anderen Riech- und/oder Aromastoffen deren Natürlichkeit, Frische, Fülle, (Strahl)Kraft und/oder Ausstrahlung abzurunden.

Eine weitere alternative bevorzugte Ausgestaltung der vorliegenden Erfindung betrifft die Verwendung einer Verbindung der Formel (II), wobei X eine - CHO, oder eine -CH₂OH Gruppe darstellt, am Ort einer der gestrichelt dargestellten Linien mindestens eine Doppelbindung vorliegt und der 3-Isopentyl- bzw. 3-Isopentenyl-Rest in ortho-, meta-, oder para-Stellung zur X-Gruppe am Ring verbunden ist, als Riech- und/oder Aromastoff.

Besonders bevorzugt ist hierbei die Verwendung der folgenden Molekülvarianten als Riech- und/oder Aromastoff:

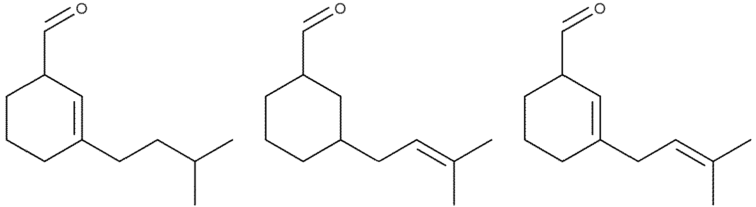

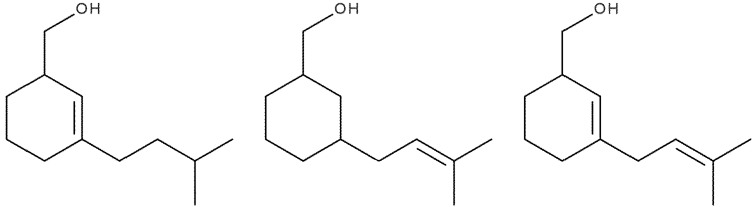

Die Molekülvarianten sind jeweils bevorzugt mit dem meta-Substituenten dargestellt, der Substituent kann auch jeweils in der ortho- oder para-Position gebunden sein, wie beispielsweise bei: 2-Isopentylcyclohex-2-ene-1-carbaldehyde, 4-Isopentylcyclohex-2-ene-1-carbaldehyde, 2-(3-Methylbut-2-enyl)cyclohexancarbaldehyde, 4-(3-Methyl-but-2-enyl)cyclohexancarbaldehyde, [2-(3-Methylbut-2-enyl)cyclohexyl]methanol, [4-(3-Methylbut-2-enyl)cyclohexyl]methanol, (2-Isopentylcyclohex-2-en-1-yl)methanol oder (4-Isopentylcyclohex-2-en-1-yl)methanol. Sämtliche oben gezeigten Beispielformeln sind daher auch mit ortho- oder para-Substituent bevorzugt.

Die erfindungsgemäßen bzw. erfindungsgemäß einzusetzenden Verbindungen der Formel (I), der Formel (la), der Formel (Ib), der Formel (Ic), und/oder der Formel (II) können in beliebiger optisch aktiver Form, wie auch als beliebige Mischung von Stereoisomeren, vorliegen, beispielsweise als Diastereomerengemisch oder als Racemat.

Eine bevorzugte Variante der vorliegenden Erfindung betrifft Verbindungen der Formel (I), der Formel (la), der Formel (Ib), der Formel (Ic), und/oder der Formel (II), in allen Formen ihrer cis- oder trans-Isomere (E/Z Isomere) an der 2-, 3-, oder 4-Position, sowie deren Mischungen.

In einem dritten Aspekt betrifft die Erfindung die Verwendung einer Verbindung der Formel (I), der Formel (la), der Formel (Ib), der Formel (Ic), und/oder der Formel (II) nach erfindungsgemäßen Varianten, als Rosen-Noten Riechstoff.

In einem vierten Aspekt betrifft die Erfindung die Verwendung einer Verbindung der Formel (I), der Formel (la), der Formel (Ib), der Formel (Ic), und/oder der Formel (II) zum Vermitteln, Modifizieren und/oder Verstärken einer oder mehrerer Geruchsnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig.

Eine bevorzugte Ausgestaltung der vorliegenden Erfindung betrifft die Verwendung einer Verbindung der Formel (I), der Formel (la), der Formel (Ib), der Formel (Ic), und/oder der Formel (II) nach erfindungsgemäßen Varianten zum Vermitteln einer, zweier oder mehrerer Geruchsnoten ausgewählt aus der Gruppe bestehend aus den Noten grün, frisch, fruchtig und holzig.

Vorzugsweise betrifft die vorliegende Erfindung die Verwendung einer Verbindung der Formel (I), der Formel (la), der Formel (Ib), der Formel (Ic), und/oder der Formel (II) nach erfindungsgemäßen Varianten als Riech- und/oder Aromastoff,
i) mit Rosen-Note
   und/oder
ii) zum Vermitteln, Modifizieren und/oder Verstärken einer oder mehrerer Geruchsnoten ausgewählt aus der Gruppe bestehend aus den Noten grün, krautig, frisch, fruchtig, holzig, süß, erdig, fettig, metallisch und balsamisch, wobei vorzugsweise zumindest einer der Noten grün und/oder fruchtig vermittelt, modifiziert und/oder verstärkt wird.

In einem fünften Aspekt betrifft die Erfindung einen Riech- und/oder Aromastoff umfassend mindestens eine Verbindung der Formel (I), der Formel (la), der Formel (Ib), der Formel (Ic), und/oder der Formel (II) nach erfindungsgemäßen Varianten.

Die Verbindungen der Formel (I), der Formel (la), der Formel (Ib), der Formel (Ic), und/oder der Formel (II) eignen sich wegen ihrer olfaktorischen Eigenschaften vorzüglich für den Einsatz in Riech- und/oder Aromastoffmischungen und/oder Riech- und/oder Aromastoffkompositionen. Eine oder mehrere Verbindungen der Formel (I), der Formel (la), der Formel (Ib), der Formel (Ic), und/oder der Formel (II) können mit einer Vielzahl weiterer Riech- und/oder Aromastoffe kombiniert und in zahlreichen unterschiedlichen Produkten und Artikeln verwendet werden. Besonders vorteilhaft lassen sich die Verbindungen der Formel (I), der Formel (la), der Formel (Ib), der Formel (Ic), und/oder der Formel (II) mit anderen Riech- und/oder Aromastoffen in verschiedenen, unterschiedlichen Mengenverhältnissen zu erfindungsgemäßen Riech- und/oder Aromastoffmischungen und/oder Riech-und/oder Aromastoffkompositionen kombinieren.

In einer weiteren Variante betrifft die Erfindung eine Riech- und/oder Aromastoffmischung mit mindestens einem Riech- und/oder Aromastoff gemäß dem fünften Aspekt und/oder 2-(3-Methylbutyl)cyclohexan-1-ol und/oder 3-(3-Methylbutyl)-cyclohexan-1-ol und/oder 4-(3-Methylbutyl)cyclohexan-1-ol , weiterhin umfassend einen oder mehrere zusätzliche Riechstoffe und/oder Aromastoffe, wobei der bzw. die zusätzlichen bzw. ein, mehreren oder sämtlichen Riechstoffe und/oder Aromastoffe ausgewählt ist bzw. sind aus einer Gruppe bestehend aus: Extrakten aus natürlichen Rohstoffen, vorzugsweise Etherische Öle, Concretes, Absolues, Resine, Resinoide, Balsame, Tinkturen und/oder Einzel-Riechstoffen, vorzugsweise aus der Gruppe der (1) Kohlenwasserstoffe; (2) Aliphatischen Alkohole; (3) Aliphatischen Aldehyde und deren Acetale; (4) Aliphatischen Ketone und deren Oxime; (5) Aliphatischen schwefelhaltigen Verbindungen; (6) Aliphatischen Nitrile; (7) Ester von aliphatischen Carbonsäuren; (8) Acyclischen Terpenalkohole; (9) Acyclischen Terpenaldehyde und -ketonen; (10) Cyclischen Terpenalkohole; (11) Cyclischen Terpenaldehyde und - ketone; (12) Cyclischen Alkohole; (13) Cycloaliphatischen Alkohole; (14) Cyclischen und cycloaliphatischen Ether; (15) Cyclischen und makrocyclischen Ketone; (16) Cycloaliphatischen Aldehyde; (17) Cycloaliphatischen Ketone; (18) Ester cyclischer Alkohole; (19) Ester cycloaliphatischer Alkohole; (20) Ester cycloaliphatischer Carbonsäuren; (21) Araliphatischen Alkohole; (22) Ester von araliphatischen Alkoholen und aliphatischen Carbonsäuren; (23) Araliphatischen Ether; (24) Aromatischen und araliphatischen Aldehyde; (25) Aromatischen und araliphatischen Ketone; (26) Aromatischen und araliphatischen Carbonsäuren und deren Ester; (27) Stickstoffhaltigen aromatischen Verbindungen; (28) Phenole, Phenylether und Phenylester; (29) Heterocyclischen Verbindungen; (30) Lactone; sowie deren Gemische.

In einem sechsten Aspekt betrifft die Erfindung eine Riech- und/oder Aromastoffmischung umfassend mindestens eine Verbindung der Formel (III), 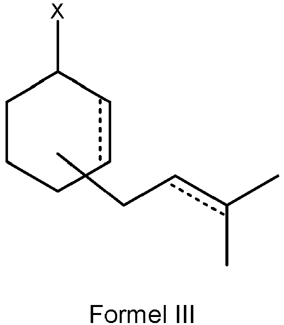 wobei
X eine -CHO, eine -OH, oder eine -CH₂OH Gruppe darstellt,
am jeweiligen Ort einer der gestrichelt dargestellten Linien jeweils eine Einfachbindung oder eine Doppelbindung vorliegt, und wobei der 3-Isopentyl- oder 3-Isopent-2-enyl-Rest in ortho-, meta-, oder para-Stellung zur X-Gruppe mit dem Ring verbunden ist und mindestens einen weiteren Riechstoff.

Die erfindungsgemäßen bzw. erfindungsgemäß einzusetzenden Verbindungen der Formel (I), der Formel (la), der Formel (Ib), der Formel (Ic), der Formel (II) und/oder der Formel (III) werden im Rahmen der erfindungsgemäßen Verwendung üblicherweise in einer sensorisch wirksamen Menge eingesetzt, d.h. in einer Gesamtmenge, in der sie eine sensorische Wirkung entfalten.

Vorzugsweise werden die erfindungsgemäßen bzw. erfindungsgemäß einzusetzenden Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol zusammen mit anderen Riechstoffen eingesetzt.

Solche Riech- und/oder Aromastoffmischungen und/oder Riech- und/oder Aromastoffkompositionen können in üblicher Weise hergestellt werden, beispielsweise durch einfaches Vermischen oder Homogenisieren der Bestandteile. Bei diesen weiteren Riech- und/oder Aromastoffen kann es sich um beliebige weitere Riech-und/oder Aromastoffe handeln.

In einer bevorzugten erfindungsgemäßen Riech- und/oder Aromastoffmischung und/oder Riechstoff und/oder Aromastoffkomposition liegt das Gewichtsverhältnis der Gesamtmenge an Verbindungen der Formel (I), der Formel (I), der Formel (la), der Formel (Ib), der Formel (Ic), der Formel (II) und/oder der Formel (III) vorzugsweise in einer der vorstehend als bevorzugt angegebenen Ausgestaltungen, zu der Gesamtmenge an weiteren Riech- und/oder Aromastoffen im Bereich von 1 : 1000 bis 1 : 0.5.

Insbesondere durch Kombination der Verbindungen der oben genannten Formeln, und insbesondere der Formel (I) und Formel (III), in erfindungsgemäßen Varianten, vorzugsweise in einer der als bevorzugt angegebenen Ausgestaltungen, mit einem oder mehreren weiteren Riech- und/oder Aromastoffen (vorzugsweise mit holzigem, fruchtigem und/oder blumigem Geruch oder Geschmack) lassen sich interessante neue Riech- und/oder Aromastoffmischungen und/oder Riech- und/oder Aromastoffkompositionen herstellen. Auf diese Weise lassen sich Mischungen mit besonders interessanten, natürlichen, neuen und originellen Noten kreieren. Riech-und/oder Aromastoffe, die als (b)-Komponente, wie oben beschrieben, einer erfindungsgemäßem Riech- und/oder Aromastoffkomposition geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt: Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (*E*,*Z*)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (*E*)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (*E*,*Z*)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (*E*)-2-Hexenal; (*Z*)-4-Heptenal; 2,6-Dimethyl-5-heptenal; 10-Undecenal; (*E*)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(*Z*)-3-hexen; 1-(1-Methoxy-propoxy)-(*E*)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (*E*)- und (*Z*)-3-Hexenylformiat; Ethylacetoacetat; Isoamylacetat; 3,5,5-Trimethylhexylacetat; 3-Methyl-2-butenyl-acetat; (*E*)-2-Hexenylacetat; (*E*)- und (*Z*)-3-Hexenylacetat; Octylacetat; 3-Octylacetat; 1-Octen-3-ylacetat; Ethylbutyrat; Butylbutyrat; Isoamylbutyrat; Hexylbutyrat; (*E*)- und (*Z*)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(*E*,*Z*)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal; 7-Methoxy-3,7-dimethyloctanal; 2,6,10-Trimethyl-9-undecenal; Geranylaceton; sowie die Dimethyl-und Diethylacetale von Geranial, Neral,
der cyclischen Terpenalkohole wie z. B. Menthol; Isopulegol; α-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; α-lonon; β-Ionon; α-n-Methylionon; β-n-Methylionon; α-Isomethylionon; β-Isomethyl-ionon; α-Iron; β-Damascenon; 1-(2,4,4-Trimethyl-2-cyclohexen-1-yl)-2-buten-1-on; 1,3,4,6,7,8a-Hexa-hydro-1,1,5,5-tetramethyl-2*H*-2,4a-methano-naphthalen-8(5*H*)-on; 2-Methyl-4-(2,6,6-trimethyl-1-cyclohexen-1-yl)-2-butenal; Nootkaton; Dihydronootkaton; 4,6,8-Megastigmatrien-3-on; α-Sinensal; β-Sinensal; acetyliertes Cedernholzöl (Methylcedrylketon);
der cyclischen Alkohole wie z.B. 4-*tert*-Butylcyclohexanol; 3,3,5-Trimethylcyclohexanol; 3-Isocamphylcyclohexanol; 2,6,9-Trimethyl-*Z*2,*Z*5,*E*9-cyclododecatrien-1-ol; 2-Isobutyl-4-methyltetrahydro-2*H*-pyran-4-ol;
der cycloaliphatischen Alkohole wie z.B. α-3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethyl-cyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy)cyclo-dodecan; α-Cedrenepoxid; 3a,6,6,9a-Tetramethyldodecahydro-naphtho[2,1b]furan; 3a-Ethyl-6,6,9a-trimethyldodecahydronaphtho[2,1b]fura; 1,5,9-Trimethyl-13-oxabi-cyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-*tert*-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetra-methylcyclohexanon; 4-*tert*-Pentylcyclo-hexanon; 5-Cyclohexadecen-1-on; 6,7-Dihydro-1,1,2,3,3-pentamethyl-4(5*H*)-indanon; 8-Cyclohexadecen-1-on; 9-Cyclo-heptadecen-1-on; Cyclopentadecanon; Cyclohexadecanon;
der cycloaliphatischen Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphta-lenylmethylketon; Methyl-2,6,10-trimethyl-2,5,9-cyclododecatrienylketon; tert-Butyl-(2,4-dimethyl-3-cyclohexen-1-yl)keton;
der Ester cyclischer Alkohole wie z.B. 2-tert-Butylcyclohexylacetat; 4*-tert-*Butylcyclohexylacetat; 2-*tert*-Pentylcyclohexylacetat; 4-*tert*-Pentyl-cyclo-hexyl-acetat; 3,3,5-Trimethylcyclohexylacetat; Decahydro-2-naphthylacetat; 2-Cyclo-pentylcyclopentylcrotonat; 3-Pentyltetrahydro-2*H*-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5 bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; *cis*- und *trans*-Methyldihydrojasmonat; *cis*- und *trans-*Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenyl-pentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenyl-ethylisovalerianat; 1-Phenylethylacetat; α-Trichlormethylbenzylacetat; α,α-Dimethylphenylethylacetat; α,α-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydro-indeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-*tert*-Butyl-phenyl)-propanal; Zimtaldehyd; α-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-*tert*-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon; (3-Methyl-2-benzofuranyl)ethanon; Benzophenon; 1,1,2,3,3,6-Hexamethyl-5-indanylmethylketon; 6-*tert*-Butyl-1,1-di-methyl-4-indanylmethylketon; 1-[2,3-dihydro-1,1,2,6-tetramethyl-3-(1-methylethyl)-1*H*-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenyl-glycidat;
der stickstoffhaltigen aromatischen Verbindungen wie z.B. 2,4,6-Trinitro-1,3-dimethyl-5-*tert*-butylbenzol; 3,5-Dinitro-2,6-dimethyl-4-*tert*-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-*tert*-butyl-phenyl)propanal oder 2,4-Dimethyl-3-cyclohexencarbaldehyd; 6-Isopropyl-chinolin; 6-Isobutylchinolin; 6-*sec-*Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; Isoeugenylmethylether; Thymol; Carvacrol; Diphenylether; β-Naphthylmethylether; β-Naphthylethylether; β-Naphthyliso-butylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2*H*-furan-3-on; 2-Ethyl-4-hydroxy-5-methyl-2*H*-furan-3-on; 3-Hydroxy-2-methyl-4*H*-pyran-4-on; 2-Ethyl-3-hydroxy-4*H*-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

In einem siebten Aspekt betrifft die vorliegende Erfindung eine Riech-und/oder Aromastoffkomposition umfassend:
(a)-Komponente: eine oder mehrere Verbindungen der Formel (I), der Formel (la), der Formel (Ib), der Formel (Ic), der Formel (II) und/oder der Formel (III) in erfindungsgemäßen Varianten, und/oder 2-(3-Methylbutyl)cyclohexan-1-ol und/oder 3-(3-Methylbutyl)cyclohexan-1-ol und/oder 4-(3-Methylbutyl)cyclohexan-1-ol, sowie
(b)-Komponente: einen oder mehrere, vorzugsweise zwei, drei, vier, fünf, sechs, sieben, acht, neun, zehn oder mehr, weitere Riech- und/oder Aromastoffe.

In einer weiteren Variante betrifft die vorliegende Erfindung daher eine Riech- und/oder Aromastoffkomposition umfassend:
a) eine oder mehrere Verbindungen der Formel (I) nach Anspruch 1 bis 3 und/oder 2-(3-Methylbutyl)cyclohexan-1-ol und/oder 3-(3-Methylbutyl)cyclohexan-1-ol und/oder 4-(3-Methylbutyl)cyclohexan-1-ol und/oder sowie
b) einen oder mehrere, vorzugsweise zwei, drei, vier, fünf, sechs, sieben, acht, neun, zehn oder mehr, weitere Riech- und/oder Aromastoffe;
   (i) wobei der oder die weiteren Riech- und/oder Aromastoffe einen holzigen, fruchtigen und/oder blumigen Geruch und/oder Geschmack vermitteln, oder
   (ii) wobei der oder die weiteren Riech- und/oder Aromastoffe einen anderen Geruch vermitteln.

In einer bevorzugten Ausgestaltung einer erfindungsgemäßen Riech-und/oder Aromastoffkomposition sind die erfindungsgemäßen bzw. erfindungsgemäß zu verwendenden Verbindungen der Formel (I), der Formel (la), der Formel (Ib), der Formel (Ic), der Formel (II), und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol vorzugsweise mit einem oder mehreren, besonders bevorzugt mit zwei, drei, vier, fünf oder mehr, blumigen und/oder fruchtigen weiteren Riech- und/oder Aromastoffen kombiniert.

In einer weiteren bevorzugten Ausgestaltung betrifft die vorliegende Erfindung Riech- und/oder Aromastoffkompositionen und/oder 2-(3-Methylbutyl)cyclohexan-1-ol und/oder 3-(3-Methylbutyl)cyclohexan-1-ol und/oder 4-(3-Methylbutyl)cyclohexan-1-ol, die einen, zwei, drei, vier, fünf oder mehrere Riech-und/oder Aromastoffe umfasst, die eine blumige und/oder fruchtige Geruchsnote vermitteln.

Dabei wird durch die erfindungsgemäße bzw. erfindungsgemäß zu verwendende Verbindung der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol vorteilhafterweise (zumindest teilweise) eine geruchliche Verstärkung der blumigen Riech- und/oder Aromastoffe erreicht.

Blumige Riech- und/oder Aromastoffe, mit denen die erfindungsgemäßen bzw. die erfindungsgemäß zu verwendenden Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol (insbesondere in erfindungsgemäßen Riech-und/oder Aromastoffkompositionen) vorteilhaft kombiniert werden können, werden vorzugsweise ausgewählt aus der Gruppe bestehend aus:
Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol^{®}), 4-tert-Butyl-α-methyldihydrozimtaldehyd (Lilial^{®}), cis-Hexahydrocuminylalkohol (Mayol^{®}), 3-[4-(1,1-Dimethylethyl)phenyl]propanal (Bourgeonal^{®}), 2,2-Dimethyl-3-(3-methylphenyl)-propanol (Majantol^{®}), 3-Methyl-3-(3-methylbenzyl)-butan-2-ol, 2-Isobutyl-4-methyl-tetrahydro-2*H*-pyran-4-ol (Florosa^{®}), 2-Methyl-3-(3,4-methylendioxyphenyl)propanal (Heliofolal^{®}), 4-(4-Hydroxy-4-methylpentyl)-3-cyclohexencarbaldehyd (Lyral^{®}), 4-(Octahydro-4,7-methano-5*H*-inden-5-yliden-butanal (Dupical^{®}), Vernaldehyd, 4-(4-Methyl-3-penten-1-yl)-3-cyclohexencarbaldehyd (Vertomugal^{®}), Octahydro-5-(4-methoxybutyliden)-4,7-methano-1*H*-inden (Mugoflor^{®}), 2,6-Dimethyl-2-heptanol (Freesiol^{®}), 1-Ethyl-1-methyl-3-phenylpropanol (Phemec^{®}), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol, Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion^{®}, Hedione^{®} high cis), 2-Heptylcyclopentanon (Projasmon P), cis-Jasmon, Dihydrojasmon, Zimtalkohol (3-Phenyl-2-propen-1-ol), Dihydrozimtalkohol (3-Phenylpropanol), 2-Methyl-4-phenyl-1,3-dioxolan (Jacinthaflor^{®}) und Dihydromyrcenol (2,6-Dimethyl-7-octen-2-ol).

In einem achten Aspekt betrifft die vorliegende Erfindung eine Riech-und/oder Aromastoffkomposition, wobei die weiteren ein oder mehrere Riechstoffe und/oder Aromastoffe ausgewählt ist bzw. sind aus der Gruppe bestehend aus: Ethyl 2-cyclopent-2-en-1-ylacetat, 1-Cyclohexylethyl (E)-but-2-enoat, (2-Cyclopentylcyclopentyl) (E)-But-2-enoat, Allyl 3-cyclohexylpropanoat, Allylhexanoat, 1,3-Dimethylbutyl (E)-but-2-enoat, 1,3-Dimethylbut-3-enyl 2-methylpropanoat, und/oder (E)-1-(2,6,6-Tri-methylcyclohex-2-en-1-yl)but-2-en-1-on.

In einer bevorzugten Ausgestaltung einer erfindungsgemäßen Riech-und/oder Aromastoffkomposition sind die erfindungsgemäßen bzw. erfindungsgemäß zu verwendenden Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol vorzugsweise mit einem oder mehreren, besonders bevorzugt mit zwei, drei, vier, fünf oder mehr weiteren Riech- und/oder Aromastoffen kombiniert, wobei die Riech- und/oder Aromastoffe ausgewählt ist aus folgender Gruppe: Ethyl 2-cyclopent-2-en-1-ylacetat, 1-Cyclohexylethyl (E)-but-2-enoat, (2-Cyclopentylcyclopentyl) (E)-But-2-enoat, Allyl 3-cyclohexylpropanoat, Allylhexanoat, 1,3-Dimethylbutyl (E)-but-2-enoat, 1,3-Dimethylbut-3-enyl 2-methylpropanoat, (E)-1-(2,6,6-Trimethylcyclohex-2-en-1-yl)but-2-en-1-on.

Zudem sind erfindungsgemäße bzw. erfindungsgemäß zu verwendende Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol vorteilhafterweise dazu geeignet, insbesondere fruchtige Riech- und/oder Aromastoffe, hinsichtlich ihres Geruchs zu verstärken.

Fruchtige Riech- und/oder Aromastoffe, mit denen die erfindungsgemäßen bzw. die erfindungsgemäß zu verwendenden Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol vorteilhaft kombiniert werden können, und die demnach besonders bevorzugte (weitere) Riechstoffe einer erfindungsgemäßen Riech- und/oder Aromastoffkompositionen sind, sind vorzugsweise ausgewählt aus der Gruppe bestehend aus:
2-Methyl-buttersäureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersäureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, γ-Undecalacton, γ-Nonalacton, Hexanal, 3Z-Hexenal, n-Decanal, n-Dodecanal, Citral, Vanillin, Ethylvanillin, Maltol, Ethylmaltol und deren Mischungen.

In einer weiteren Variante ist in der Riech- und/oder Aromastoffkomposition das Gewichtsverhältnis der Gesamtmenge an Verbindungen der Formel (I) oder zuvor genanntem Erstriechstoff zu der Gesamtmenge an weiteren Riech- und/oder Aromastoffen, vorzugsweise im Bereich von 1: 1000 bis 1: 0.5.

In einer bevorzugten erfindungsgemäßen Riech- und/oder Aromastoffmischungen und/oder -kompositionen liegt die Gesamtmenge an Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol im Bereich von 0.0001 bis 99.9 Gew.-%, vorzugsweise im Bereich von 0.001 bis 99.5 Gew.-%, besonders bevorzugt im Bereich von 0.01 bis 99 Gew.-%, jeweils bezogen auf die Gesamtmasse der Riech- und/oder Aromastoffmischungen und/oder Riech-und/oder Aromastoffkompositionen.

In weiteren bevorzugten erfindungsgemäßen Riech- und/oder Aromastoffmischungen und/oder Riech- und/oder Aromastoffkompositionen liegt die Gesamtmenge an Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol im Bereich von 0.01 bis 90 Gew.-%, vorzugsweise im Bereich von 0.05 bis 80 Gew.-%, weiter bevorzugt im Bereich von 0.1 bis 70 Gew.-%, besonders bevorzugt im Bereich von 0.25 bis 50 Gew.-%, insbesondere bevorzugt im Bereich von 0.5 bis 40 Gew.-%, am meisten bevorzugt im Bereich von 0.75 bis 25 Gew.-%, jeweils bezogen auf die Gesamtmasse der Riech- und/oder Aromastoffmischungen und/oder Riech- und/oder Aromastoffkompositionen.

In einer bevorzugten erfindungsgemäßen Riech- und/oder Aromastoffmischungen und/oder Riech- und/oder Aromastoffkompositionen reicht die eingesetzte Gesamtmenge an der oder den Verbindungen der Formel (I), der Formel (la), der Formel (Ib), der Formel (Ic), der Formel (II) und/oder der Formel (III) in erfindungsgemäßen Varianten, um den Geruch der weiteren Riech- und/oder Aromastoffe in Richtung grün, krautig, frisch, fruchtig, blumig, holzig, süß, erdig, fettig, metallisch und balsamisch, vorzugsweise in Richtung grün und/oder fruchtig, bevorzugt in Richtung Rose zu modifizieren, zu verstärken und/oder eine oder mehrere der genannten Noten zu vermitteln.

Sofern eine oder mehrere Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol hauptsächlich eingesetzt wird bzw. werden, um einer Riech- und/oder Aromastoffmischungen und/oder Riech- und/oder Aromastoffkompositionen mehr Frische, (Aus)Strahlung, Abrundung, Harmonie und/oder Natürlichkeit zu verleihen und/oder bestimmte (durch weitere Riech- oder Aromastoffe bereits vorhandene) Noten zu verstärken, liegt die Gesamtmenge der Komponente (a) vorzugsweise vergleichsweise niedrig und besonders bevorzugt im Bereich von 0.01 bis 5 Gew.-%, bevorzugt im Bereich von 0.1 bis 2 Gew.-%, bezogen auf die Gesamtmenge der Riech- und/oder Aromastoffmischungen und/oder Riech- und/oder Aromastoffkompositionen. Sofern innerhalb der bevorzugten Konzentrationsbereiche eine vergleichsweise niedrige Konzentration gewählt wird, kommt es in Abhängigkeit von den weiteren Komponenten der jeweiligen Komposition in manchen Fällen noch nicht zu der Vermittlung der oben angegebenen Eigengeruchsnoten.

Die vorstehend mit Blick auf die erfindungsgemäßen Verwendungen beschriebenen bevorzugten Ausgestaltungen der Erfindung gelten entsprechend auch für erfindungsgemäße Riech- und/oder Aromastoffmischungen und/oder Riech- und/oder Aromastoffkompositionen sowie erfindungsgemäße parfümierte Artikel, insbesondere die Angaben zu bevorzugten Gewichtsverhältnissen.

Die erfindungsgemäße bzw. erfindungsgemäß einzusetzenden Verbindung(en) der Formel (I), der Formel (la), der Formel (Ib), der Formel (Ic), und/oder der Formel (II) insbesondere die im Rahmen dieser Erfindung als bevorzugt bzw. besonders bevorzugt gekennzeichneten Verbindungen der Formel (I), der Formel (la), der Formel (Ib), der Formel (Ic), der Formel (II) und/oder der Formel (III) verfügen über einen sehr komplexen und vielfältigen Geruchseindruck. Dieser kann sonst meist nur durch Mischungen mehrerer Komponenten (wie beispielsweise etherischen Ölen, oder Gewürzmischungen) erreicht werden.

Über ihre primären, nämlich geruchlichen, Eigenschaften hinaus verfügen die Verbindungen der Formel (I), der Formel (la), der Formel (Ib), der Formel (Ic), der Formel (II) und/oder der Formel (III) zusätzlich über positive Sekundäreigenschaften, insbesondere ein gutes Haftungsvermögen und eine hohe Substantivität im Vergleich zu Riech- und/oder Aromastoffen mit ähnlichen Geruchseigenschaften, sowie eine hohe Stabilität in bestimmten Medien und Zubereitungen und eine hohe Ausgiebigkeit.

In einem achten Aspekt betrifft die Erfindung ein Verfahren zum Modifizieren und/oder Verstärken (Boosten) eines Geruchs mit einer, mehreren oder sämtlichen der Noten fruchtig und/oder holzig und umfasst die folgenden Schritte:
(a) Bereitstellen einer Verbindung der Formel (I), der Formel (la), der Formel (Ib), der Formel (Ic), der Formel (II) und/oder der Formel (III) nach einem der vorangehenden Ansprüche 1 bis 3 und/oder einer/mehrerer erfindungsgemäßen/ erfindungsgemäßer Riechstoffmischung(en) und/oder -komposition(en);
(b) Vermischen einer sensorisch wirksamen Menge dieses Stoffes oder dieser Stoffe mit einer Mischung an einem oder mehreren Riech- und/oder Aromastoff(en) mit einer/mehreren/sämtlichen der Noten fruchtig und oder holzig, die ausreicht, um in der fertigen Zubereitung eine fruchtig und/oder holzige Geruchsnote hervorzurufen, sensorisch zu modifizieren und/oder zu verstärken.

Die erfindungsgemäßen bzw. erfindungsgemäß zu verwendenden Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol können zudem die Intensität einer erfindungsgemäßen Riech- und/oder Aromastoffmischung verstärken und das Gesamtbild der Mischung geruchlich abrunden und können eingesetzt werden, um einer Riech- und/oder Aromastoffkomposition mehr Fülle, Frische, (Strahl)Kraft, Ausstrahlung, Glanz, Abrundung, Harmonie und/oder Natürlichkeit zu verleihen.

Eine oder mehrere Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol können insbesondere eingesetzt werden, um einer Riech-und/oder Aromastoffkomposition Frische, (Aus)Strahlung, Abrundung, Harmonie und/oder Natürlichkeit zu verleihen und/oder vorhandene Geruchsnoten zu verstärken.

In einem neunten Aspekt betrifft die vorliegende Erfindung die Verwendung einer oder mehrere Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol zum Versehen von Haaren und/oder Haut oder von textilen Fasern mit einem Duft (hinsichtlich der dabei bevorzugt vermittelten Geruchsnoten sei auf die obigen Ausführungen verwiesen), wobei die Verbindung(en) der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)-cyclohexan-1-ol und/oder 4-(3-Methylbutyl)cyclohexan-1-ol vorzugsweise in Kombination mit einem Tensid oder einer Tensidmischung eingesetzt wird bzw. werden.

In einem neunten Aspekt betrifft die vorliegende Erfindung die Verwendung der Verbindungen der Formel (I), der Formel (la), der Formel (Ib), der Formel (Ic), der Formel (II) und/oder der Formel (III) und/oder 2-(3-Methylbutyl)cyclohexan-1-ol und/oder 3-(3-Methylbutyl)cyclohexan-1-ol und/oder 4-(3-Methylbutyl)cyclohexan-1-ol erfindungsgemäße Riech- und/oder Aromastoffmischungen und/oder Riech- und/oder Aromastoffkompositionen als Mittel zum Erhöhen der Substantivität und/oder Retention einer Riech- und/oder Aromastoffmischung und/oder Riech- und/oder Aromastoffkomposition und/oder als Fixateur.

Die sensorischen Eigenschaften von Verbindungen der Formel (I) und deren Eignung als Riech- und/oder Aromastoff wurden bisher nicht beschrieben und zeigen überraschende Vorteile, insbesondere auch eine erhöhte Substantivität.

Die nachfolgende Tabelle 1 enthält die sensorischen, insbesondere die geruchlichen, Beschreibungen von einigen erfindungsgemäßen bzw. erfindungsgemäß einzusetzenden Verbindungen der Formel (I).

Maßgeblich ist hierbei stets die dargestellte Strukturformel der Verbindungen der Formeln (I).

**Tabelle 1**

| **Nr.:** | **Struktur** | **Sensorische Beschreibung** | **Substantivität** |
|---|---|---|---|
| 11 | 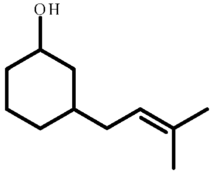 | rosig, fettig, grün | gute Haftung t_{1/2}= 3d nach 48h noch riechend |
| 12 | 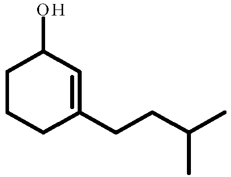 | rosig, grün | gute Haftung t_{1/2}= 3d nach 48h noch riechend |
| 5 | 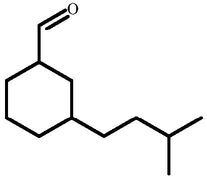 | rosig, aldehydisch, blumig, grün, Citronellol | sehr gut haftend t_{1/2}= 3d nach 48h noch stark riechend |
| 6 | 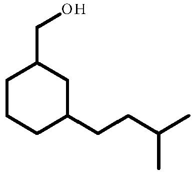 | stark rosig, natürlich | sehr gut haftend t_{1/2}= 3d nach 48h noch stark riechend |
| 7 | 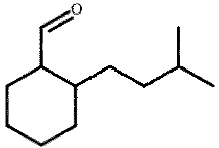 | aldehydisch, blumig, grün | sehr gut haftend t_{1/2}= 3d nach 48h noch stark riechend |
| 8 | 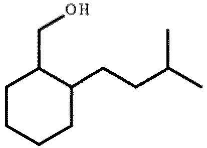 | Graphfrucht, rosig, fruchtig | sehr gut haftend t_{1/2}= 3d nach 48h noch stark riechend |
| 9 | 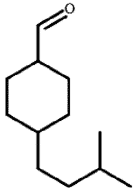 | ledrig, marine, Graphfrucht, Rhabarber ozonartig, blumig, grün | sehr gut haftend t_{1/2}= 3d nach 48h noch stark riechend |
| 10 | 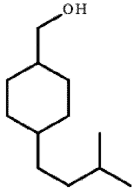 | blumig, rosig, grasartig | sehr gut haftend t_{1/2}= 3d, dry down nach 48h noch stark riechend |

Besonders bevorzugt sind weiterhin Riechstoffkombinationen oder Riechstoffkompositionen. Hierbei wurde insbesondere eine außerordentliche Erhöhung der fruchtigen Note einiger Riechstoffe durch Kombination mit weiteren Zweit-Riechstoffen erzielt. Die nachfolgende Tabelle 2 enthält die sensorischen, insbesondere die geruchlichen, Beschreibungen von 4-(3-Methylbutyl)cyclohexan-1-ol (Symrose) mit Zweit- bzw. Kombinationsriechstoffen.

**Tabelle 2**

| **Nr.:** | **Zweitriechstoffname** | **Struktur** | **Dosierung von Symrose mit Zweitriechstoff** | **Geruchsgemisch** |
|---|---|---|---|---|
| **21** | **Sultanene** | 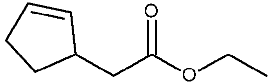 | 0,5-1 % | weicher, runder, fruchtiger, weniger metallisch |
| **22** | **Aprifloren** | 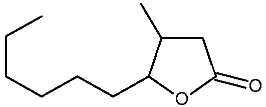 | 0,5-1 % | Reduktion der unangenehmen fetti- gen Noten |
| **23** | **Datilat** | 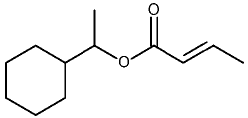 | 0,5-1 % | mehr Impact, mehrtrocken fruchtiger in Richtung Pflaume |
| **24** | **Buccoxime** | 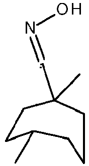 | 0,5-1 % | weicher, runder, weniger technisch |
| **25** | **Vertacetal Coeur** | 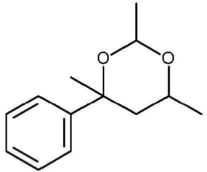 | 0,5-1 % | weniger unangenehm muffig, natürlicher |
| **26** | **Oxanthia 50% in TEC** | 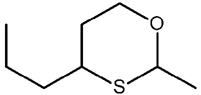 | 0,5-1 % | weicher, runder, fruchtiger, weniger metallisch |
| **27** | **Cassiffix** | 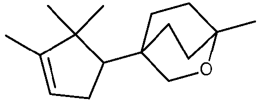 | 0,5-1 % | stärker fruchtiger, runder und natürlicher |
| **28** | **Amarocit** | 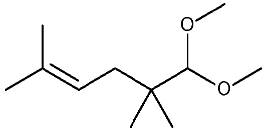 | 0,5-1 % | mehr fruchtiger und grüner, natürlicher |
| **29** | **Isoamylacetat** | 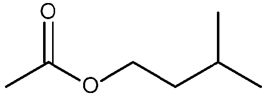 | 0,5-1 % | weicher, runder, natürlicher, weniger metallisch |
| **30** | **Fragolane** | 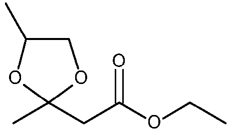 | 0,5-1 % | weicher, runder, weniger metallisch |
| **31** | **Ethyl Methyl Butyrat-2** | 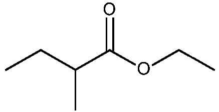 | 0,5-1 % | fruchtiger, runder |
| **32** | **Pyroprunat** | 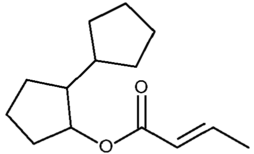 | 0,5-1 % | fruchtig-süßer, mehr reife Frucht |
| **33** | **Cassix 150** | 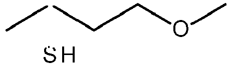 | 0,5-1 % | stärker fruchtiger, runder und natürlicher |
| **34** | **Allylcyclohexylpropionat** | 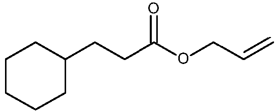 | 0,5-1 % | stärker fruchtiger |
| **35** | **Allylcapronat** | 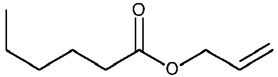 | 0,5-1 % | stärker fruchtiger |
| **36** | **Frutinat** | 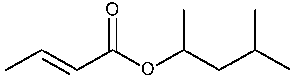 | 0,5-1 % | mehr Impact, saftiger-fruchtiger, natürlicher |
| **37** | **Isopentyrate** | 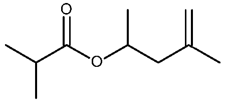 | 0,5-1 % | mehr Impact, mehr Strahlung, natürlicher, süßer |
| **38** | **Damascon alpha** | 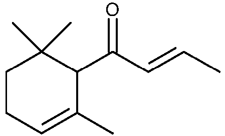 | 0,5-1 % | weicher, runder, fruchtiger, weniger metallisch |

In einer bevorzugten Variante betrifft die Erfindung Riechstoffkompositionen und Riechstoffkombinationen, umfassend: eine oder mehrere Verbindungen der Formel (I) nach erfindungsgemäßen Varianten und/oder 2-(3-Methylbutyl)cyclohexan-1-ol und/oder 3-(3-Methylbutyl)cyclohexan-1-ol und/oder 4-(3-Methylbutyl)cyclohexan-1-ol und mindestens einen Riech- und/oder Aromastoffmischung in einer sensorisch wirksamen Menge, wobei der/die Riech- und/oder Aromastoffmischung ausgewählt ist aus folgender Gruppe: Ethyl 2-cyclopent-2-en-1-ylacetat, 1-Cyclohexylethyl (E)-but-2-enoat, (2-Cyclopentylcyclopentyl) (E)-But-2-enoat, Allyl 3-cyclohexylpropanoat, Allylhexanoat, 1,3-Dimethylbutyl (E)-but-2-enoat, 1,3-Dimethylbut-3-enyl 2-methylpropanoat, und/oder (E)-1-(2,6,6-Trimethylcyclohex-2-en-1-yl)but-2-en-1-on.

Durch die Kombination von 4-(3-Methylbutyl)cyclohexan-1-ol (Symrose) mit anderen zweiten Riechstoffen wird der Geruchseindruck der Riechstoffe verstärkt. Sie wirken oftmals fruchtiger und natürlicher und bekommen daher einen leicht abgewandelten Charakter. Fehlgerüche wie metallische Noten werden nicht mehr wahrgenommen.

Bevorzugt wird Symrose oder anderen in der Erfindung genannte Verbindungen mit folgenden Zweitriechstoffen kombiniert: Sultanene, Aprifloren, Datilat, Buccoxime, Vertacetal Coeur, Oxanthia 50% in TEC, Cassiffix, Amarocit, Fragolane, Pyroprunat, Cassix 150, Allylcyclohexylpropionat, Allylcapronat, Frutinat, Isopentyrate, Damascon alpha.

Auch die folgenden Varianten und Grundriechstoffe der Verbindungen 3-(3-Methylbutyl)cyclohexan-1-on (2) oder 3-(3-Methylbutyl)cyclohexan-1-ol (3) geben mit den in Tabelle 2 genannten Zweit- bzw. Kombinationsriechstoffen einen verstärkt synergistischen fruchtigen Geruch, der teils schon von dem Grundriechstoff kommt aber durch Kombination besonders verstärkt wird.

In einem zehnten Aspekt betrifft die Erfindung parfümierte oder aromatisierte Artikel, umfassend:
i) eine oder mehrere Verbindungen der Formel (I) nach erfindungsgemäßen Varianten und/oder 2-(3-Methylbutyl)cyclohexan-1-ol und/oder 3-(3-Methylbutyl)cyclohexan-1-ol und/oder 4-(3-Methylbutyl)cyclohexan-1-ol und/oder eine erfindungsgemäße Riech- und/oder Aromastoffmischung und/oder -komposition in einer sensorisch wirksamen Menge,
ii) einen, zwei, drei, vier, fünf, sechs, sieben, acht, neun, zehn oder mehr weitere Riech- oder Aromastoffe, wobei vorzugsweise einer, mehrere oder sämtliche der weiteren Riechstoffe einen fruchtigen und/holzigen Geruch vermitteln,
iii) einen oder mehrere weitere Zusatz-, Hilfs- und/oder Wirkstoffe, vorzugsweise zwei, drei, vier, fünf oder mehr Zusatz-, Hilfs- und/oder Wirkstoffe. Es versteht sich dabei, dass die weiteren Zusatz-, Hilfs- und/oder Wirkstoffe keine Riech- und Geschmackstoffe sind.

In einer bevorzugten Ausführungsform betrifft die Erfindung parfümierte oder aromatisierte Artikel, umfassend: eine oder mehrere Verbindungen der Formel (I) nach erfindungsgemäßen Varianten und/oder 2-(3-Methylbutyl)cyclohexan-1-ol und/oder 3-(3-Methylbutyl)cyclohexan-1-ol und/oder 4-(3-Methylbutyl)cyclohexan-1-ol und mindestens einen Riech- und/oder Aromastoffmischung in einer sensorisch wirksamen Menge, wobei der/die Riech- und/oder Aromastoffmischung ausgewählt ist aus folgender Gruppe: Ethyl 2-cyclopent-2-en-1-ylacetat, 1-Cyclohexylethyl (E)-but-2-enoat, (2-Cyclopentylcyclopentyl) (E)-But-2-enoat, Allyl 3-cyclohexylpropanoat, Allylhexanoat, 1,3-Dimethylbutyl (E)-but-2-enoat, 1,3-Dimethylbut-3-enyl 2-methylpropanoat, und/oder (E)-1-(2,6,6-Trimethylcyclohex-2-en-1-yl)but-2-en-1-on.

Zusatz-, Hilfs- und/oder Wirkstoffe, welche ein erfindungsgemäßer parfümierter oder aromatisierter Artikel zusätzlich zu einer oder mehreren Verbindungen der Formel (I), der Formel (la), der Formel (Ib), der Formel (Ic), und/oder der Formel (II) und/oder 2-(3-Methylbutyl)cyclohexan-1-ol und/oder 3-(3-Methylbutyl)-cyclohexan-1- und/oder 4-(3-Methylbutyl)cyclohexan-1-ol und/oder einer erfindungsgemäßen Riech- und/oder Aromastoffmischung und/oder Riech- und/oder Aromastoffkomposition (wie nachfolgend definiert) enthalten kann, sind vorzugsweise ausgewählt aus der Gruppe bestehend aus:

Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, AntischuppenMittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildner, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, (weitere) Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-β-dicarbonylverbindungen, vorzugsweise die in WO 2005/107692 genannten, α-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, fette Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Farbstoffe und farbschützende Mittel sowie Pigmente, vorzugsweise die in WO 2005/123101 genannten, Antikorrosiva, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, haarwachstumsmodulierende Mittel (haarwachstumsfördend oder haarwachstumshemmend), vorzugsweise die in EP 2168570 und EP 2193785 genannten oder Silikone und Silikonderivate, vorzugsweise die in WO 2008/046676 genannten.

Eine bevorzugte Ausgestaltung der vorliegenden Erfindung betrifft parfümierte oder aromatisierte Artikel, umfassend
- eine oder mehrere Verbindungen der Formel (I), der Formel (la), der Formel (Ib), der Formel (Ic), und/oder der Formel (II) und/oder 2-(3-Methylbutyl)cyclohexan-1-ol und/oder 3-(3-Methylbutyl)cyclohexan-1-ol und/oder 4-(3-Methylbutyl)cyclohexan-1-ol und/oder eine Riech- und/oder Aromastoffmischung und/oder Riech- und/oder Aromastoffkomposition, vorzugsweise in einer der vorstehend als bevorzugt angegebenen Ausgestaltungen, in einer sensorisch wirksamen Menge, die ausreicht, eine oder mehrere der Geruchsnoten grün, krautig, frisch, fruchtig, blumig, holzig, süß, erdig, fettig, metallisch und balsamisch, zu vermitteln, zu modifizieren und/oder zu verstärken, bevorzugt zu vermitteln und/oder zu verstärken,
- einen, zwei, drei, vier, fünf, sechs, sieben, acht, neun, zehn oder mehr weitere Riech- und/oder Aromastoffe, wobei vorzugsweise einer, zwei, drei oder mehrere der weiteren Riech- und/oder Aromastoffe einen holzigen, fruchtigen und/oder blumigen Geruch vermitteln,
- zwei, drei, vier, fünf oder mehr Zusatz-, Hilfs- und/oder Wirkstoffe, wobei bevorzugt ein, zwei, drei oder mehr Zusatz-, Hilfs- und/oder Wirkstoffe ausgewählt sind aus der Gruppe bestehend aus Konservierungsmitteln, anorganischen Salzen, Chelatbildnern, Tensiden, haut- und/oder haarpflegenden Mitteln, Enzymen Emulgatoren, Fetten, fetten Ölen, Wachsen, Fettalkoholen, Silikonen, Silikonderivaten und Wasser.

Eine weitere bevorzugte Ausgestaltung der vorliegenden Erfindung betrifft erfindungsgemäß parfümierte oder aromatisierte Artikel ausgewählt aus der Gruppe bestehend aus Wasch- und Reinigungsmitteln, Hygiene- oder Pflegeprodukten, insbesondere im Bereich der Körper- und Haarpflege, der Kosmetik und des Haushalts.

Da eine erfindungsgemäße Riech- und/oder Aromastoffkomposition zur Parfümierung oder Aromatisierung von Artikeln geeignet ist, betrifft die Erfindung in einer weiteren bevorzugten Ausführungsform einen parfümierten oder aromatisierten Artikel, umfassend:
- eine erfindungsgemäße Riechstoffkomposition, vorzugsweise in einer der als bevorzugt gekennzeichneten Ausgestaltungen, vorzugsweise in einer sensorisch wirksamen Menge, und
- einen oder mehrere weitere Zusatz-, Hilfs- und/oder Wirkstoffe, vorzugsweise zwei, drei, vier, fünf oder mehr Zusatz-, Hilfs- und/oder Wirkstoffe, vorzugsweise zwei, drei, vier, fünf oder mehr der oben als bevorzugt angegebenen Zusatz-, Hilfs- und/oder Wirkstoffe.

Ein alternative erfindungsgemäß bevorzugte Variante der vorliegenden Erfindung betrifft erfindungsgemäße parfümierte oder aromatisierte Artikel, die ausgewählt werden aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und - lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

Eine weitere bevorzugte Variante der vorliegenden Erfindung betrifft parfümierte oder aromatisierte Artikel, in denen die Gesamtmenge an Verbindungen der Formel (I), der Formel (la), der Formel (Ib), der Formel (Ic), und/oder der Formel (II) und/oder 2-(3-Methylbutyl)cyclohexan-1-ol und/oder 3-(3-Methylbutyl)cyclohexan-1-ol und/oder 4-(3-Methylbutyl)cyclohexan-1-ol und/oder eine Riech- und/oder Aromastoffmischungen und/oder Riech- und/oder Aromastoffkompositionen nach der Erfindung, vorzugsweise in einer der vorstehend als bevorzugt angegebenen Ausgestaltungen, im Bereich von 0,00001 bis 10 Gew.-%, vorzugsweise im Bereich von 0.0001 bis 5 Gew.-%, besonders bevorzugt im Bereich von 0.001 bis 2 Gew.-%, am meisten bevorzugt 0.005 bis 1 Gew.-%, jeweils bezogen auf die Gesamtmasse des parfümierten oder aromatisierten Artikels, liegt.

Die Erfindung betrifft in einem elften Aspekt die Verwendung einer erfindungsgemäßen Riech- und/oder Aromastoffkomposition, vorzugsweise in einer der als bevorzugt angegebenen Ausgestaltungen,
(A) als Riech- und/oder Aromastoffkomposition
   (a)
      (i) mit Rose-Note, und/oder
      (ii) zum Vermitteln, Modifizieren und/oder Verstärken einer oder mehrerer Geruchsnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig, und/oder
   (b)
      (i) mit fruchtiger und/oder mit blumiger Note, und/oder
      (ii) zum Vermitteln, Modifizieren und/oder Verstärken einer oder mehrerer Geruchs- und/oder Geschmacksnoten ausgewählt aus der Gruppe bestehend aus den Noten holzig, fruchtig und/oder blumig, und/oder
   (c) mit blumiger, fruchtiger, Rosennote
   und/oder
(B) als Bestandteil eines parfümierten oder aromatisierten Artikels.

Erfindungsgemäße Riech- und/oder Aromastoffkompositionen, welche eine oder mehrere Verbindungen der Formel (I), der Formel (la), der Formel (Ib), der Formel (Ic), und/oder der Formel (II) und/oder 2-(3-Methylbutyl)cyclohexan-1-ol und/oder 3-(3-Methylbutyl)cyclohexan-1-ol und/oder 4-(3-Methylbutyl)cyclohexan-1-ol enthalten, können in flüssiger Form, unverdünnt oder mit einem Lösungsmittel verdünnt zur Parfümierung eingesetzt werden. Bevorzugte Lösungsmittel hierfür sind Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin und Diacetin.

Des Weiteren können erfindungsgemäße Riech- und/oder Aromastoffkompositionen, welche Verbindungen der Formel(I), der Formel (la), der Formel (Ib), der Formel (Ic), und/oder der Formel (II) und/oder 2-(3-Methylbutyl)cyclohexan-1-ol und/oder 3-(3-Methylbutyl)cyclohexan-1-ol und/oder 4-(3-Methylbutyl)cyclohexan-1-ol enthalten, an einem Trägerstoff adsorbiert sein, der sowohl für eine feine Verteilung der Riech- und/oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulosebasierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Erfindungsgemäße Riech- und/oder Aromastoffkompositionen, die Verbindungen enthalten, können auch mikroverkapselt, sprühgetrocknet, als Einschluss-Komplexe oder als Extrusions-Produkte (d. h. erfindungsgemäße Artikel) vorliegen und in dieser Form z. B. einem zu parfümierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riech- und/oder Aromastoffkompositionen zu erfindungsgemäßen Artikeln kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- und/oder Aromastoffkompositionen können beispielsweise durch Sprühtrocknung einer die Riech- und/oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech-und/oder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riech- und/oder Aromastoffkompositionen mit einem geeigneten wachsartigen Stoff und durch Extrusion mit nachfolgender Erstarrung, gegebenenfalls in einem geeigneten Lösungsmittel, z.B. Isopropanol, erhalten werden.

Verbindungen der Formel (I), der Formel (la), der Formel (Ib), der Formel (Ic), und/oder der Formel (II) und erfindungsgemäße Riechstoffkompositionen, die eine oder mehrere Verbindungen der Formel (I) und/oder 2-(3-Methylbutyl)cyclohexan-1-ol und/oder 3-(3-Methylbutyl)cyclohexan-1-ol und/oder 4-(3-Methylbutyl)cyclohexan-1-ol enthalten, können in konzentrierter Form, in Lösungen oder in oben beschriebener modifizierter Form verwendet werden für die Herstellung von erfindungsgemäßen parfümierten oder aromatisierten Artikeln wie z. B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und - lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 Gew.-% Hilfsstoffe, bevorzugt natürliche oder künstliche Polysaccharide und/oder Pflanzengummen wie modifizierte Stärken oder Gummi Arabicum enthalten.

Die Erfindung betrifft in einem zwölften Aspekt ein Verfahren zum Vermitteln, Modifizieren und/oder Verstärken einer oder mehrerer Geruchsnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig, bevorzugt einer Rosen-Note, mit folgenden Schritten
- Bereitstellen einer oder mehrerer Verbindungen der Formel (I) wie oben definiert, vorzugsweise in einer der vorstehend als bevorzugt angegebenen Ausgestaltungen, oder einer erfindungsgemäßen Riech- und/oder Aromastoffkomposition wie oben definiert, vorzugsweise in einer der vorstehend als bevorzugt angegebenen Ausgestaltungen,
- Bereitstellen eines Artikels,
- In Kontakt bringen des Artikels mit einer sensorisch wirksamen Menge von der oder den Verbindungen der Formel (I) bzw. von der Riech- und/oder Aromastoffkomposition wie oben definiert.

Ein entsprechendes bevorzugtes erfindungsgemäßes Verfahren ist dabei ein Verfahren, bei dem nach dem in Kontakt bringen (i) ein parfümierter oder aromatisierter Artikel resultiert oder (ii) aus dem dann vorliegenden Erzeugnis hergestellt wird, der ausgewählt ist aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

Die Erfindung betrifft in einem dreizehnten Aspekt ein Verfahren zur Herstellung einer erfindungsgemäßen bzw. erfindungsgemäß zu verwendenden Verbindungen der Formel (I), der Formel (la), der Formel (Ib), der Formel (Ic), und/oder der Formel (II). Die Synthese kann mittels an sich bekannter Reaktionen und Verfahren erfolgen. Zum Beispiel kann das Keton (2) mittels einer 1,4-Grignard-Reation hergestellt werden aus der käuflichen Verbindung 2-Cyclohex-3-en (Verbindung 1). Die anschließende Reduktion des Ketons (2) ergibt den Alkohol (3) (siehe nachfolgendes Schema 1).

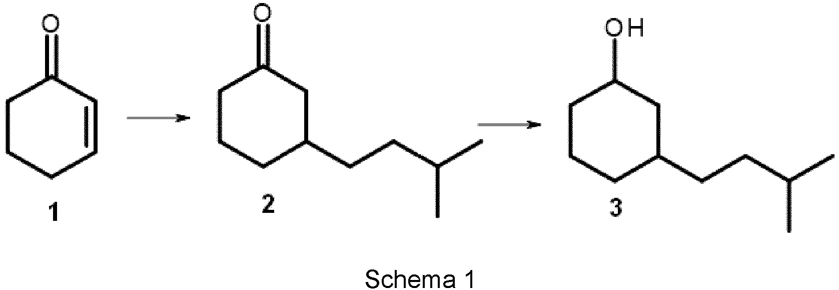

Die Erfindung betrifft in einem vierzehnten Aspekt ein Verfahren zur Herstellung einer Verbindung (I) umfassend zumindest die Schritte (i), (ii) und (iii), dadurch gekennzeichnet, dass ausgehend von 2-, oder 3-, oder 4-Isopentlyclohexanon durch
i) Wittig-Reaktion 1-Isopentyl-2-(methoxymethylen)cyclohexan (Verbindung 4a), 1-Isopentyl-3-(methoxymethylen)cyclohexan (Verbindung 4b), oder 1-Isopentyl-4-(methoxymethylen)cyclohexan (Verbindung 4c) erhalten wird,
ii) anschließende Enolspaltung zum Aldehyd (Verbindung 5 a, b, c) führt
iii) und durch optional daran anschließende Reduktion der Alkohol (Verbindung 6 a, b, c) erhalten wird (siehe nachfolgendes Schema 2).

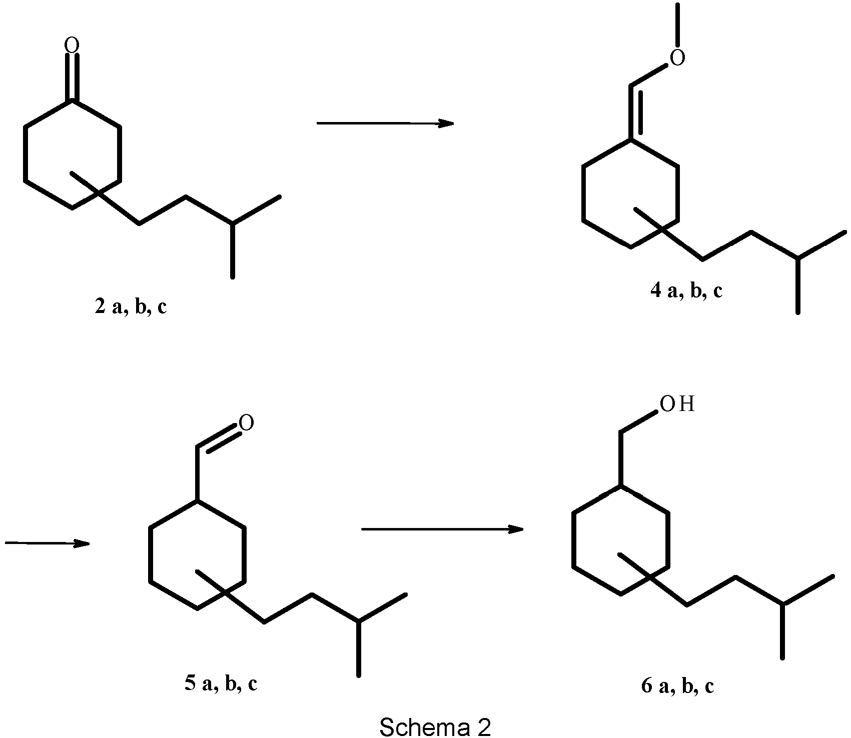

Nachfolgend wird die Erfindung anhand von Beispielen näher erläutert. Sofern nicht anders angegeben, beziehen sich alle Angaben auf das Gewicht.

Bei der Angabe der Dezimalstellen wurde entgegen der deutschen Rechtschreibung ein Punkt statt einem Komma gesetzt.

Verwendete Abkürzungen: DPG: Dipropylenglycol, TEC: Triethylcitrat, MTBE: Methyl-tert-butylether, THF: Tetrahydrofuran, LAH: Lithiumaluminiumhydrid, RT: Raumtemperatur.

### Ausführliche Beschreibung der Erfindung

Die Erfindung wird nachstehend anhand von Ausführungsbeispielen beschrieben. Wie nachfolgend näher erläutert wird, lassen sich die erfindungsgemäßen Verbindungen der Formel (I) in folgender, unten beschriebener Weise synthetisieren. Weiterhin ist zu beachten, dass die IUPAC Nomenklatur von der bisher verwendeten generischen Bezeichnung abweichen kann.

### Beispiel 1: 3-Isopentylcyclohexanon (2)

Herstellung des Grignard-Reagenz: Magnesiumspäne (16.5 g, 0.687 mol) im Mörser leicht zerreiben, und in 300 ml THF im Kolben vorlegen, eine Spatelspitze Jod zugeben, unter Ruckfluss erhitzen (Lösung muss sich entfärben) erst dann 1/10 von 3-Methylbutylbromid (93.8 g, 0.621 mol) zu tropfen lassen, nach Beginn der Bildung des Grignard-Reagenzes das restliche 3-Methylbutylbromid in 150 ml THF zu tropfen lassen, den Ansatz 1h refluxieren, und anschließend Ansatz abkühlen lassen.

Die Grignard-Lösung wird über eine Kanüle in eine gut gerührte, auf -5°C gekühlte Suspension von Kupferiodid (8.0 g, 0.042 mol) in 300 ml THF überführt. Nach Auflösen des Kupferiodids auf -20°C abkühlen lassen und Cyclohex-2-en-1-on (34 g, 0.354 mol) zu tropfen und bei -20°C 4 Stunden (h) nachrühren lassen dann auf RT auftauen und über Nacht nachrühren.

Aufarbeitung: Ansatz in 600 ml eiskalte gesättigte Ammoniumchlorid-Lösung geben, 300 ml MTBE zugeben. Phasentrennung, dreimal mit 100 ml MTBE extrahieren und die vereinigten organischen Phasen 2x mit 200 ml gesättigter (ges.) NaCl Lösung waschen, trocknen und einengen. Das Rohprodukt (60.0 g) wurde an einer 15 cm Vigreux-kolone im Vakuum fraktioniert destilliert.
Ausbeute: 52.0 g (87%)
¹H-NMR (400 MHz, CDCl₃) δ 2.42 (ddt, *J* = 13.8, 4.0, 1.9 Hz, 1H), 2.35 (dddt, *J* = 14.1, 5.0, 3.6, 1.5 Hz, 1H), 2.25 (dddd, *J* = 14.1, 12.2, 6.1, 1.2 Hz, 1H), 2.04 (dddd, *J* = 13.8, 8.0, 6.3, 3.9 Hz, 1H), 2.00 (ddt, *J* = 13.7, 11.6, 1.1 Hz, 1H), 1.96-1.84 (m, 1H), 1.79-1.70 (m, 1H), 1.70-1.58 (m, 1H), 1.50 (dp, *J* = 13.2, 6.6 Hz, 1H), 1.40-1.24 (m, 3H), 1.23-1.14 (m, 2H), 0.87 (d, *J* = 6.6 Hz, 6H);
¹³C-NMR (101 MHz, CDCl₃) δ 212.16 (C=O), 48.31 (CH_{2-ring}), 41.55 (CH_{2-ring}), 39.38 (CH_{-ring}), 35.91 (CH₂), 34.35 (CH₂), 31.35 (CH_{2-ring}), 28.08 (CH-(CH₃)₂, 25.32 (CH_{2-ring}), 22.58 (CH-(CH₃)₂, 22.57(CH-(CH₃)₂.

### Beispiel 2: 3-Isopentylcyclohexanol (3)

LAH (2.48 g, 0.065 mol) wird in 200 ml THF bei RT vorgelegt. Bei 0-10 °C wird Verbindung **2** (10.0 g) in 70 ml THF vorsichtig hinzugetropft. Es wird bei RT 30 min nachgerührt.

Aufarbeitung: Ansatz auf 300 ml Eiswasser geben (exotherm, H₂ Entwickelung). Ansatz mit 150 ml MTBE und anschließend mit 150 ml 25%iger Schwefelsäure versetzen bis sich der gebildete Niederschlag (Al(OH)₃) langsam auflöst, Phasen trennen, wässrige Phase 2x mit je 50 ml MTBE extrahieren, vereinigte organische Phasen 1x mit 100 ml Soda-Lösung und 1x mit 100 ml gesättigter NaCl-Lsg. waschen, über Na₂SO₄ trocknen und einengen. Das Rohprodukt (10.0 g) wird mithilfe einer Vigreux-kolonne im Vakuum bei (60 °C/500-10 mbar) fraktioniert destilliert.
Ausbeute: 9.5 g (94%)
*trans*-Isomer: NMR: ¹H-NMR (400 MHz, CDCl₃) δ 4.09-4.01 (m, 1H), 1.78-1.57 (m, 5H), 1.56-1.45 (m, 3H), 1.25 (ddd, *J* = 13.3, 10.5, 2.9 Hz, 1H), 1.21-1.15 (m, 4H), 1.04-0.92 (m, 1H), 0.86 (d, *J* = 6.6 Hz, 6H)
¹³C-NMR (101 MHz, CDCl₃) δ 67.08 (CH), 39.97 (CH₂), 36.41 (CH₂), 34.26 (CH₂), 33.61 (CH₂), 32.35 (CH₂), 31.97 (CH), 28.36 (CH), 22.81 (CH₃), 22.81 (CH₃), 20.21 (CH₂).
*cis*-Isomer: NMR: ¹H-NMR (400 MHz, CDCl₃) δ 3.55 (tt, *J* = 10.8, 4.2 Hz, 1H), 2.03-1.93 (m, 2H), 1.76 (dp, *J* = 12.9, 3.2 Hz, 1H), 1.69-1.61 (m, 1H), 1.55-1.42 (m, *J* = 6.5 Hz, 1H), 1.33-1.17 (m, 6H), 1.16-1.07 (m, 1H), 0.93-0.83 (m, 1H), 0.87 (d, *J* = 6.6 Hz, 6H), 0.77 (tdd, *J* = 12.6, 10.9, 3.5 Hz, 1H)
¹³C-NMR (101 MHz, CDCl₃) δ 71.14 (CH), 42.93 (CH₂), 36.90 (CH), 36.35 (CH₂), 36.06 (CH₂), 34.91(CH₂), 32.35 (CH₂), 28.36 (CH), 24.33 (CH₂), 22.83 (CH₃) 22.81 (CH₃).

### Beispiel 3: (3 E/Z)-1-Isopentyl-3-(methoxymethylene) cyclohexan (4)

Methoxymethyltriphenylphosphoniumchlorid (84.2 g, 0.246 mol) in 500 ml THF vorlegen und auf 0 °C abkühlen lassen, Kalium-*tert*-Butylat (112 g, 0.40 mol) bei 0 bis 5 °C portionsweise hinzugeben. 30 Minuten nachrühren lassen und anschließend 3-Isopentylcyclohexanone (27.5 g, 0.164 mol) in 100 ml THF langsam in die Reaktionslösung tropfen lassen, die Temperatur dabei unter 10 °C halten. 30 Minuten bei < 10 °C nachrühren lassen und danach langsam im Verlauf von 22 h auf RT aufwärmen.

Aufarbeitung: Ansatz mit H₂O quenchen, 3x mit 1000 ml Ethylacetat extrahieren, die vereinigten organischen Phasen mit 200 ml ges. NaCl-Lösung waschen, trocknen und einengen (60 °C/500-10 mbar). Das Rohprodukt (96.2 g) wurde an einem Kugelrohr destilliert. (150 °C, 0.8 mbar).
Ausbeute: 36.2 g (Quantitativ)
¹H-NMR (400 MHz, CDCl₃) δ 5.75 (d, *J* = 1.7 Hz, 1H), 5.74 (t, *J* = 2.0 Hz, 1H), 3.53 (s, 6H), 2.75-2.67 (m, 1H), 2.66-2.59 (m, 1H), 2.06 (dt, *J* = 13.0, 2.3 Hz, 1H), 2.02-1.91 (m, 1H), 1.91-1.80 (m, 1H), 1.80-1.69 (m, 4H), 1.70-1.55 (m, 1H), 1.49 (dddd, *J* = 12.8, 10.4, 6.3, 2.6 Hz, 2H), 1.43 (s, 2H), 1.40-1.11 (m, 13H), 1.10-0.94 (m, 2H), 0.94-0.81 (m, 12H).
¹³C-NMR (101 MHz, CDCl₃) δ 138.95, 138.94 ᵢₛₒₘₑᵣ, 117.98, 117.93 ᵢₛₒₘₑᵣ, 59.28, 59.28 ᵢₛₒₘₑᵣ, 39.44 ᵢₛₒₘₑᵣ, 38.21 ᵢₛₒₘₑᵣ, 37.14, 37.43, 37.14 ᵢₛₒₘₑᵣ, 36.27, 36.24 ᵢₛₒₘₑᵣ, 34.68, 34.51 ᵢₛₒₘₑᵣ, 33.19, 31.38, 28.32, 28.30 ᵢₛₒₘₑᵣ, 28.25, 28.23 ᵢₛₒₘₑᵣ, 26.93 ᵢₛₒₘₑᵣ, 22.70, 22.67ᵢₛₒₘₑᵣ, 22.65, 22.63ᵢₛₒₘₑᵣ.

### Beispiel 4: 3-isopentylcyclohexancarbaldehyd (5)

In einen 1l Rührwerk wurde Verbindung 4 (35.0 g, 0.178 mol) in THF (400 ml) und 10% HCI-Lösung (96 ml) vorgelegt, danach wurden die Edukte nacheinander zugegeben und die Mischung auf Siedetemperatur erwärmt, 1h unter Rückfluss erhitzen.

Aufarbeitung: Ansatz mit Wasser quenchen und 3x mit MTBE extrahieren. Die vereinigten organischen Phasen einmal mit H₂O waschen, trocknen und eindampfen (60 °C/500-10 mbar). Das Rohprodukt (31.0 g) wurde an Vigreux-Kolone im Vakuum fraktioniert destilliert.
Ausbeute: 31.0 g (95%)
*trans*-Isomer: NMR: ¹H-NMR (400 MHz, CDCl₃) δ 9.60 (d, *J* = 1.57 Hz, 1H), 2.23 (ttd, *J* = 12.17, 3.52, 1.62 Hz, 1H), 1.98 (m_{c}, 3H), 1.75 (tq, *J* = 14.30, 3.34, 1.72 Hz, 1H), 1.71 (m_{c}, 1H), 1.64 (m_{c}, 3H), 1.20 (m_{c}, 5H), 1.14 (*J* = 3.70 Hz, 1H), 0.86 (d, *J* = 6.73 Hz, 6H).
¹³C-NMR (101 MHz, CDCl₃) δ 204.83, 50.64, 37.13, 36.31, 36.05, 35.05, 33.58, 28.22, 26.12, 25.44, 22.63, 22.63.

### Beispiel 5: (3-Isopentylcyclohexyl) methanol (6)

Unter Stickstoff (Apparatur gründlich spülen), LAH (3.2 g, 0.085 mol) wird in 200 ml THF bei RT vorgelegt. Bei 0-10 °C wird 3-Isopentylcyclohexancarbaldehyde (14.0 g, 0.077 mol) in 70ml THF vorsichtig hinzugetropft. Es wird bei RT 30 min nachgerührt. Aufarbeitung: Ansatz auf 300 ml Eiswasser geben (exotherm, H₂ Entwickelung). Ansatz mit 150 ml MTBE versetzen anschließend 150 ml 25%iger Schwefelsäure zugeben bis sich der gebildete Niederschlag (Al(OH)₃) langsam auflöst, Phasen trennen, wässrige Phase 2x mit je 50 ml MTBE extrahieren, vereinigte organische Phasen 1x mit 100 ml Soda-Lsg. und 1x mit 100 ml gesättigter NaCl-Lsg. waschen, über Na₂SO₄ trocknen, eindampfen (60 °C/500-10 mbar). Das Rohprodukt (13.3 g) wurde an einer Kugelrohr-Destille destilliert. (90 °C, 1.0 mbar).
Ausbeute: 12.7 g (90%)
¹H-NMR (600 MHz, CDCl₃) δ 3.46 (dd, *J* = 10.5, 6.3 Hz, 1H), 3.44 (dd, *J* = 10.6, 6.3 Hz, 1H), 1.82-1.71 (m, 3H), 1.56-1.45 (m, 2H), 1.45-1.36 (m, 2H), 1.31-1.21 (m, 2H), 1.21-1.12 (m, 3H), 0.88 (d, *J* = 6.8 Hz, 1H), 0.86 (d, *J* = 6.6 Hz, 6H), 0.85-0.77 (m, 2H), 0.57 (q, *J* = 11.9 Hz, 1H);
¹³C-NMR (151 MHz, CDCl₃) δ 68.93, 40.60, 37.50, 36.18, 35.46, 33.41, 32.77, 29.59, 28.54, 25.82, 22.71, 22.68.

### Beispiel 6: 1-isopentyl cyclohexan carbaldehyd (7)

Analog zur Verbindung 3 wurde Verbindung 7 hergestellt.

1H-NMR (400 MHz, CDCl3) δ 9.81 (d, J = 1.3 Hz, 1H), 9.55 (d, J = 4.0 Hz, 1H), 2.45 (dtd, J = 6.8, 3.9, 1.9 Hz, 1H), 2.01 (ddt, J = 10.0, 10.1, 3.9 Hz, 1H), 1.93 - 1.81 (m, 4H), 1.80 - 1.66 (m, 3H), 1.65 - 1.59 (m, 4H), 1.59 - 1.51 (m, 3H), 1.51 - 1.43 (m, 2H), 1.42 - 1.30 (m, 5H), 1.29 - 1.20 (m, 4H), 1.19 - 1.08 (m, 3H), 0.92 (d, J = 6.6 Hz, 3H), 0.87 (d, J = 6.6 Hz, 3H), 0.86 (d, J = 6.5 Hz, 3H), 0.84 (d, J = 6.5 Hz, 3H).

13C-NMR (101 MHz, CDCl3) δ 206.13, 205.71, 70.63, 61.38, 55.81, 52.19, 51.07, 42.00, 41.96, 41.75, 37.26, 36.94, 36.72, 36.44, 35.70, 32.44, 30.34, 29.32, 28.73, 28.19, 28.14, 27.03, 26.52, 26.11, 25.27, 25.01, 24.77, 24.70, 24.21, 23.80, 23.69, 22.87, 22.71, 22.68, 22.62, 22.52, 22.28, 22.13.

### Beispiel 7: (1-Isopentylcyclohexyl) methanol (8)

Analog zur Verbindung 4 wurde Verbindung 8 aus Verbindung 7 hergestellt.
Ausbeute: > 90%
1H-NMR (400 MHz, CDCl3) δ 3.71 (dd, J = 10.8, 3.1 Hz, 1H), 3.67-3.58 (m, 3H), 1.871.64 (m, 9H), 1.64 - 1.44 (m, 5H), 1.04 - 1.29 (m, 18H), 0.84 - 0.89 (12H).
13C-NMR (101 MHz, CDCl3) δ 65.83, 63.35, 44.10, 42.09, 38.29, 37.08, 37.01, 35.57, 35.46, 31.57, 30.96, 29.59, 29.59, 28.83, 28.44, 28.30, 26.21, 25.97, 25.77, 23.01, 22.87, 22.87, 22.53, 22.37.

### Beispiel 8: 4-isopentyl cyclohexan carbaldehyd (9)

Analog zur Verbindung 3 wurde Verbindung 9 hergestellt.

1H-NMR (400 MHz, CDCl3) δ 9.38 (s, 1H), 9.34 - 9.32 (m, 1H), 1.90 - 1.78 (m, 3H), 1.73 - 1.61 (m, 3H), 1.61 - 1.52 (m, 2H), 1.48 - 1.33 (m, 4H), 1.26 - 1.15 (m, 2H), 1.10 - 0.92 (m, 12H), 0.88 (d, J = 6.6Hz, 7H), 0.86 (d, J = 6.6 Hz, 7H), 0.68 - 0.53 (m, 2H).

13C-NMR (101 MHz, CDCl3) δ 203.68, 202.89, 50.52, 47.39, 37.61, 36.95, 36.57, 36.48, 35.20, 34.12, 32.20, 32.20, 29.84, 29.84, 28.59, 28.54, 26.17, 26.17, 24.31, 24.31, 22.83, 22.83, 22.83, 22.83.

### Beispiel 9: (4-Isopentylcyclohexyl) methanol (10)

Analog zur Verbindung 4 wurde Verbindung 10 aus Verbindung 9 hergestellt.
Ausbeute: > 90%
1H-NMR (400 MHz, CDCl3) δ 3.44 (d, J = 6.3 Hz, 2H), 1.78 (d, J = 9.1 Hz, 4H), 1.48 (ddt, J = 11.4, 7.5, 4.5 Hz, 1H), 1.41 (dd, J = 10.8, 5.3 Hz, 1H), 1.17 (d, J = 3.1 Hz, 4H), 1.15 (d, J = 3.5 Hz, 1H), 0.98 - 0.88 (m, 5H), 0.86 (d, J = 6.6 Hz, 6H).
13C-NMR (101 MHz, CDCl3) δ 122.66, 68.83, 67.44, 66.47, 40.17, 38.44, 38.12, 36.77 36.32, 35.43, 35.11, 34.18, 33.82, 32.74, 31.83, 29.52, 28.97, 28.86, 28.28, 28.24, 25.76, 25.38, 22.70, 22.68.

### Beispiel 10: Shampoo

Die Verbindung aus Beispiel 1 wurde in einer Dosierung von 0.5 Gew.-% in eine Shampoo-Grundmasse folgender Zusammensetzung eingearbeitet:

| | |
|---|---|
| Natrium laurylethersulfat (z.B. Texapon NSO, Fa. Cognis Deutschland GmbH) | 12% |
| Cocamidopropylbetain (z.B. Dehyton K, Fa. Cognis Deutschland GmbH) | 2% |
| Natriumchlorid | 1.4% |
| Citronensäure | 1.3% |
| Phenoxyethanol, Methyl-, Ethyl-, Butyl-, und Propylparaben | 0.5% |
| Wasser | 82.8% |

Der pH-Wert der Shampoo-Grundmasse lag bei etwa 6. Hieraus wurden 100 ml einer 20 Gew.-%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmem Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt. Geruchsbeschreibung beider Haarsträhnen: stark strahlend, rosenartig, fruchtig.

### Beispiel 11: Weichspüler

Die Parfümkomposition aus Beispiel 2 (nach Zusatz von 6 Gew.-% des Ketons aus Beispiel 1) wurde in einer Dosierung von 0.5 Gew.-% in eine Weichspüler-Grundmasse folgender Zusammensetzung eingearbeitet:

| | |
|---|---|
| Quarternäres Ammoniummethosulfat (Esterquat), ca. 90% (z.B. Rewoquat WE 18, Fa. Witco Surfactants GmbH) | 5.5% |
| Alkyldimethylbenzylammoniumchlorid, ca. 50% | 0.2% |
| (z.B. Preventol R50, Fa. Bayer AG) | |
| Farblösung, ca. 1%-ig | 0.3% |
| Wasser | 94.0% |

Der pH-Wert der Weichspüler-Grundmasse lag im Bereich von 2 bis 3. Zwei Stofflappen wurden mit 370 g einer 1%-igen wässrigen Weichspüler-Lösung (basierend auf der Weichspüler-Grundmasse, enthaltend 0.5 Gew.-% der Parfümkomposition aus Beispiel 2) in einer Linetest-Maschine im Weichspülprogramm 30 Minuten bei 20 °C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung beider Stofflappen: blumig, frisch, strahlend und holzige Aspekte mit leichten fruchtigen süßen Untertönen; abgerundeter und harmonischer Geruchseindruck.

### Beispiel 12: Waschpulver

Die Parfümölkomposition aus Beispiel 3 (nach Zusatz von 1 Gew.-%) wurde in einer Dosierung von 0.4 Gew.-% in eine Waschpulver-Grundmasse der folgenden Rezeptur eingearbeitet:

| | |
|---|---|
| Lineares Na-Alkylbenzolsulfonat | 8.8 % |
| Ethoxylierter Fettalkohol C12-18 (7 EO) | 4.7 % |
| Na-Seife | 3.2 % |
| Entschäumer DOW CORNING 2-4248S POWDERED ANTIFOAM, Silikonöl auf Zeolith als Trägermaterial | 3.9 % |
| Zeolith 4A | 28.3 % |
| Na-Carbonat | 11.6 % |
| Na-Salz eines Copolymers aus Acryl-und Maleinsäure (Sokalan CP5) | 2.4 % |
| Na-Silikat | 3.0 % |
| Carboxymethylcellulose | 1.2 % |
| Dequest 2066 | 2.8 % |
| ([[(Phosphonomethyl)imino]bis[(ethylennitrilo)-bis(methylen)]]tetrakis-phosphonsäure, Natriumsalz) Optischer Aufheller | 0.2 % |
| Na-Sulfat | 6.5 % |
| Protease | 0.4 % |
| Natrium perborat-tetrahydrat | 22.0 % |
| Tetraacetylethylendiam in | 1.0 % |

Zwei Stofflappen wurden mit 370 g einer 1%-igen wässrigen Waschpulverlauge auf Basis des 0.4 Gew.-% der Parfümölkomposition aus Beispiel 3 umfassenden Waschpulver-Grundmasse (der pH-Wert der Waschpulverlauge liegt deutlich im basischen Bereich) in einer Linetest-Maschine im Hauptwaschgang 45 Minuten bei 60 °C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: stark, strahlend, rosenartig mit natürlicher Note und fruchtigen Untertönen; abgerundeter und harmonischer Geruchseindruck.

### Beispiel 13: Parfum 1

| | |
|---|---|
| IONONE BETA 50 % DPG | 25.00 |
| FARENAL^{®} 10% DPG | 10.00 |
| FLORAZON | 1.00 |
| HEXENOL CIS-3 | 1.00 |
| HEXENYLACETAT CIS-3 | 1.00 |
| LIGUSTRAL | 5.00 |
| ALLYLAMYLGLYCOLAT | 6.00 |
| CYCLOGALBANAT^{®} | 6.00 |
| MELONAL 10% DPG | 6.00 |
| DIHYDROMYRCENOL | 32.00 |
| LINALYLACETAT | 10.00 |
| OXANTHIA 50% IN TEC 10% DPG | 3.00 |
| HEXYLACETAT | 25.00 |
| ISOAMYLACETAT | 2.00 |
| ETHYLBUTYRAT | 2.00 |
| ETHYLCAPRONAT | 2.00 |
| ALDEHYD C14 SOG | 15.00 |
| ALDEHYD C18 SOG. | 1.00 |
| DECALACTON GAMMA | 6.00 |
| APPLE RED AROMABASE | 4.00 |
| ETHYLMETHYLBUTYRAT-2 | 5.00 |
| MANZANATE | 5.00 |
| ALLYLCYCLOHEXYLPROPIONAT | 5.00 |
| ALLYLHEPTYLAT | 7.00 |
| PRUNELLA TYPE BASE | 12.00 |
| FRAMBINON^{®} | 10.00 |
| ETHYLMALTOL 1% DPG | 10.00 |
| CYCLAMENALDEHYD | 5.00 |
| CALONE 10% DPG | 6.00 |
| HELIONAL | 45.00 |
| FLORHYDRAL | 2.50 |
| FLOROSA | 25.00 |
| ETHYLLINALOOL | 50.00 |
| DIMETHYLBENZYLCARBINYLACETAT | 8.00 |
| TERPINEOL REIN | 12.00 |
| ROSENOXID | 1.00 |
| PHENYLETHYLALKOHOL | 56.00 |
| PHENOXANOL | 15.00 |
| SYMROSE^{®} | 1.00 |
| DAMASCON ALPHA | 1.00 |
| DAMASCONE DELTA | 1.00 |
| BENZYLACETAT | 10.00 |
| HEDION | 140.00 |
| AMYLSALICYLAT | 65.00 |
| UNDECAVERTOL | 2.50 |
| IONON ALPHA | 10.00 |
| ISOEUGENOLACETAT | 6.00 |
| AGRUMEX LC | 30.00 |
| SANDRANOL^{®} | 6.00 |
| EVERNYL 10% DPG | 6.00 |
| AMBROX DL 10% DPG | 3.00 |
| AMBRETTOLIDE | 7.00 |
| ETHYLENBRASSYLAT | 95.00 |
| GLOBALIDE^{®} | 50.00 |
| INDOFLOR^{®} KRIST. 10% DPG | 5.00 |
| DIPROPYLENGLYCOL | 119.00 |

### Beispiel 14: Parfum 2

| | |
|---|---|
| ALKOHOL C 6 KOSCHER | 6.00 |
| OCTANON-3 | 0.50 |
| HEXENOL CIS-3 | 12.00 |
| HEXENYLACETAT CIS-3 | 8.00 |
| VERTOCITRAL | 8.00 |
| CYCLOGALBANAT^{®} | 4.00 |
| FLOROPAL | 12.00 |
| MAGNOLAN | 10.00 |
| MELONAL | 4.00 |
| DIHYDROMYRCENOL | 40.00 |
| CITRONITRIL | 3.00 |
| OXANTHIA 50% IN TEC | 1.00 |
| THYMOL KRIST. | 4.00 |
| HEXYLACETAT | 40.00 |
| ISOAMYLACETAT 100% | 5.00 |
| PRENYLACETAT | 4.00 |
| ETHYLBUTYRAT | 5.00 |
| EXOVERT HIGH IMPACT 10% DPG | 2.00 |
| ALDEHYD C14 SOG | 15.00 |
| DECALACTON GAMMA | 5.00 |
| ETHYLMETHYLBUTYRAT-2 | 5.00 |
| MANZANATE | 3.00 |
| ALLYLCAPRONAT | 5.00 |
| ALLYLCYCLOHEXYLPROPIONAT | 25.00 |
| ALLYLHEPTYLAT | 40.00 |
| FRUITATE | 4.00 |
| ALDEHYD C16 SOG. | 2.00 |
| FRAMBINON^{®} | 10.00 |
| MUGETANOL | 10.00 |
| CYCLOHEXYLMAGNOL | 25.00 |
| FREESIOL / CORPS 119 | 10.00 |
| TETRAHYDROLINALOOL | 190.00 |
| DIMETHYLBENZYLCARBINYLACETAT | 8.00 |
| DIMETHYLBENZYLCARBINYLBUTYRAT | 10.00 |
| ROSENOXID | 5.00 |
| PHENIRAT^{®} | 30.00 |
| SYMROSE^{®} | 115.00 |
| DAMASCONE DELTA | 3.00 |
| ETHYLSAFRANAT | 3.00 |
| DELPHONE | 1.00 |
| VELOUTONE | 2.00 |
| NONADIENOL-2,6 1% DPG | 8.00 |
| UNDECAVERTOL | 8.00 |
| IRISNITRIL 10% DPG | 3.00 |
| AGRUMEX HC | 120.00 |
| ORYCLON SPEZIAL | 165.00 |
| HERBAFLORAT | 30.00 |
| RHUBOFIX | 0.50 |
| BRAHMANOL^{®} | 5.00 |
| SANDRANOL^{®} | 45.00 |
| SYNAMBRAN^{®} R 50 % IN IPM | 1.00 |
| GLOBALIDE^{®} | 8.00 |
| MACROLIDE^{®} SUPRA | 12.00 |

### Beispiel 15: Parfum 3

| | |
|---|---|
| FLOROPAL | 1.50 |
| MAGNOLAN | 12.00 |
| DIHYDROMYRCENOL | 8.00 |
| LINALYLACETAT | 25.00 |
| TERPINYLACETAT | 12.00 |
| CITRAL FF 10% DPG | 2.00 |
| CINEOL 1,4 10% DPG | 2.00 |
| ORANGE BASE COLIPA NEW | 1.00 |
| AMAROCIT^{®} | 14.00 |
| TERPINOLEN DEXTRO 10% DPG | 2.00 |
| LAVENDER OEL CLONALE CENSO | 2.00 |
| EUCALYPTOL NAT. | 6.00 |
| CARDAMOM OIL RCO | 0.80 |
| MATE SUPERESSENCE 10% DPG | 2.00 |
| BORNEOL L/ISOBORNEOL 65/35 10% DPG | 3.00 |
| KAMPFER DL 10% IPM | 12.00 |
| SULTANENE^{®} 1% DPG | 5.00 |
| HELIONAL | 5.00 |
| ETHYLLINALOOL | 45.00 |
| LINALOOLOXID | 1.00 |
| TETRAHYDROLINALOOL | 35.00 |
| DIMETHYLBENZYLCARBINYLBUTYRAT | 1.00 |
| TERPINEOL ALPHA | 0.50 |
| PHENYLETHYLACETAT | 0.50 |
| PHENYLETHYLALKOHOL | 13.00 |
| PHENOXANOL | 3.00 |
| SYMROSE^{®} | 1.00 |
| DAMASCENON TOTAL 10% DPG | 2.00 |
| GIVESCONE | 2.00 |
| HEDION | 220.00 |
| JASMON CIS 10% DPG | 8.00 |
| METHYLOCTINCARBONAT 10% DPG | 1.00 |
| UNDECAVERTOL | 3.00 |
| ALLYLIONON | 3.00 |
| IONON ALPHA | 12.00 |
| IONON BETA | 80.00 |
| IRON ALPHA | 2.00 |
| HELIOTROPIN/PIPERONAL | 4.00 |
| COUMARONE 10% IPM | 2.00 |
| KOUMALACTONE 10% TEC 1% DPG | 4.00 |
| OCTAHYDROCUMARIN | 4.00 |
| TONKABOHNEN ABS. 10% DPG | 6.00 |
| TABANON 1% DPG | 2.00 |
| ISO E SUPER | 80.00 |
| GUAJAKHOLZOEL | 10.00 |
| GLOBALIDE^{®} | 20.00 |
| MACROLIDE^{®} SUPRA | 2.00 |
| GALAXOLID PURE | 40.00 |
| DIPROPYLENGLYCOL | 128.70 |

### Beispiel 16: Parfum 4

| | |
|---|---|
| IONONE BETA REPL BY DPG | 5.00 |
| ESSIGSAEURE | 1.00 |
| ALDEHYD C10 | 1.00 |
| ALDEHYD C11 UNDECANAL | 0.50 |
| ALDEHYD C12 LAURIN | 3.00 |
| HEXENOL CIS-3 | 5.00 |
| HEXENYLACETAT CIS-3 | 5.00 |
| VERTOCITRAL | 5.00 |
| VERTACETAL | 0.50 |
| STYROLYLACETAT | 15.00 |
| STYROLYLPROPIONAT | 0.50 |
| ISOPROPYLMETHYLTHIAZOL-2,4 10% DPG | 1.00 |
| MELONAL 10% DPG | 2.00 |
| DIHYDROMYRCENOL | 25.00 |
| CITRONENOEL ITAL. | 5.00 |
| ORANGENOEL | 5.00 |
| AMAROCIT^{®} | 25.00 |
| CITRORANGE BASE COLIPA | 5.00 |
| MENTHOL L DEST. | 1.00 |
| ETHYLACETAT | 2.00 |
| HEXYLACETAT | 5.00 |
| ISOAMYLACETAT 100% | 1.00 |
| ISOBUTYLACETAT 10% DPG | 2.00 |
| JASMAPRUNAT | 2.50 |
| PRENYLACETAT | 0.50 |
| BUTYLBUTYRAT | 2.00 |
| HEXYLBUTYRAT | 3.00 |
| CAPRONSAEURE NAT. | 1.00 |
| ALDEHYD C14 SOG | 15.00 |
| DECALACTON GAMMA | 2.00 |
| ETHYLMETHYLBUTYRAT-2 | 1.50 |
| SYMFRESH^{®} NX | 25.00 |
| ALLYLCAPRONAT | 5.00 |
| ALLYLCYCLOHEXYLPROPIONAT | 8.00 |
| ALLYLHEPTYLAT | 4.00 |
| THIOMENTHANON-8,3 1% TEC | 2.00 |
| ETHYLMALTOL | 5.00 |
| PASSIONFRUIT TYPE BASE | 25.00 |
| MAJANTOL^{®} | 15.00 |
| TETRAHYDROLINALOOL | 50.00 |
| DIMETHYLBENZYLCARBINYLACETAT | 10.00 |
| ROSENOXID L | 1.00 |
| SYMROSE^{®} | 15.00 |
| DAMASCONE DELTA | 2.00 |
| BENZYLACETAT | 15.00 |
| HEDION | 150.00 |
| VELOUTONE | 1.50 |
| HEXYLSALICYLAT | 15.00 |
| UNDECAVERTOL | 2.50 |
| ISOEUGENOLACETAT | 0.50 |
| AGRUMEX LC | 25.00 |
| ORYCLON SPEZIAL | 10.00 |
| HERBYLPROPIONAT | 2.00 |
| SANDRANOL^{®} | 25.00 |
| ETHYLENBRASSYLAT | 25.00 |
| GLOBALIDE^{®} | 25.00 |
| DIPROPYLENGLYCOL | 389.50 |

### Beispiel 17: Parfum 5

| | |
|---|---|
| IONONE BETA 50 % DPG | 25.00 |
| FARENAL^{®} 10% DPG | 10.00 |
| FLORAZON | 1.00 |
| HEXENOL CIS-3 | 1.00 |
| HEXENYLACETAT CIS-3 | 1.00 |
| LIGUSTRAL | 5.00 |
| ALLYLAMYLGLYCOLAT | 6.00 |
| CYCLOGALBANAT^{®} | 6.00 |
| MELONAL 10% DPG | 6.00 |
| DIHYDROMYRCENOL | 32.00 |
| LINALYLACETAT | 10.00 |
| OXANTHIA 50% IN TEC 10% DPG | 3.00 |
| HEXYLACETAT | 25.00 |
| ISOAMYLACETAT | 2.00 |
| ETHYLBUTYRAT | 2.00 |
| ETHYLCAPRONAT | 2.00 |
| ALDEHYD C14 SOG | 15.00 |
| ALDEHYD C18 SOG. | 1.00 |
| DECALACTON GAMMA | 6.00 |
| APPLE RED AROMABASE | 4.00 |
| ETHYLMETHYLBUTYRAT-2 | 5.00 |
| MANZANATE | 5.00 |
| ALLYLCYCLOHEXYLPROPIONAT | 5.00 |
| ALLYLHEPTYLAT | 7.00 |
| PRUNELLA TYPE BASE | 12.00 |
| FRAMBINON^{®} | 10.00 |
| ETHYLMALTOL 1% DPG | 10.00 |
| CYCLAMENALDEHYD | 5.00 |
| CALONE 10% DPG | 6.00 |
| HELIONAL | 45.00 |
| FLORHYDRAL | 2.50 |
| FLOROSA | 25.00 |
| ETHYLLINALOOL | 50.00 |
| DIMETHYLBENZYLCARBINYLACETAT | 8.00 |
| TERPINEOL REIN | 12.00 |
| AMAROCIT | 1.00 |
| PHENYLETHYLALKOHOL | 56.00 |
| PHENOXANOL | 15.00 |
| 3-(3-Methylbutyl)cyclohexan-1-on (2) | 1.00 |
| DAMASCON ALPHA | 1.00 |
| DAMASCONE DELTA | 1.00 |
| BENZYLACETAT | 10.00 |
| HEDION | 140.00 |
| AMYLSALICYLAT | 65.00 |
| UNDECAVERTOL | 2.50 |
| IONON ALPHA | 10.00 |
| ISOEUGENOLACETAT | 6.00 |
| AGRUMEX LC | 30.00 |
| SANDRANOL^{®} | 6.00 |
| EVERNYL 10% DPG | 6.00 |
| AMBROX DL 10% DPG | 3.00 |
| AMBRETTOLIDE | 7.00 |
| ETHYLENBRASSYLAT | 95.00 |
| GLOBALIDE^{®} | 50.00 |
| INDOFLOR^{®} KRIST. 10% DPG | 5.00 |
| DIPROPYLENGLYCOL | 119.00 |

### Beispiel 18: Parfum 6

| | |
|---|---|
| IONONE BETA REPL BY DPG | 5.00 |
| ESSIGSAEURE | 1.00 |
| ALDEHYD C10 | 1.00 |
| ALDEHYD C11 UNDECANAL | 0.50 |
| ALDEHYD C12 LAURIN | 3.00 |
| HEXENOL CIS-3 | 5.00 |
| HEXENYLACETAT CIS-3 | 5.00 |
| VERTOCITRAL | 5.00 |
| VERTACETAL | 0.50 |
| STYROLYLACETAT | 15.00 |
| STYROLYLPROPIONAT | 0.50 |
| ISOPROPYLMETHYLTHIAZOL-2,4 10% DPG | 1.00 |
| MELONAL 10% DPG | 2.00 |
| DIHYDROMYRCENOL | 25.00 |
| CITRONENOEL ITAL. | 5.00 |
| ORANGENOEL | 5.00 |
| AMAROCIT^{®} | 25.00 |
| CITRORANGE BASE COLIPA | 5.00 |
| MENTHOL L DEST. | 1.00 |
| ETHYLACETAT | 2.00 |
| HEXYLACETAT | 5.00 |
| ISOAMYLACETAT 100% | 1.00 |
| ISOBUTYLACETAT 10% DPG | 2.00 |
| JASMAPRUNAT | 2.50 |
| PRENYLACETAT | 0.50 |
| BUTYLBUTYRAT | 2.00 |
| HEXYLBUTYRAT | 3.00 |
| CAPRONSAEURE NAT. | 1.00 |
| ALDEHYD C14 SOG | 15.00 |
| DECALACTON GAMMA | 2.00 |
| ETHYLMETHYLBUTYRAT-2 | 1.50 |
| SYMFRESH^{®} NX | 25.00 |
| ALLYLCAPRONAT | 5.00 |
| ALLYLCYCLOHEXYLPROPIONAT | 8.00 |
| ALLYLHEPTYLAT | 4.00 |
| THIOMENTHANON-8,3 1% TEC | 2.00 |
| ETHYLMALTOL | 5.00 |
| PASSIONFRUIT TYPE BASE | 25.00 |
| MAJANTOL^{®} | 15.00 |
| TETRAHYDROLINALOOL | 50.00 |
| DIMETHYLBENZYLCARBINYLACETAT | 10.00 |
| DAMASCON ALPHA | 1.00 |
| 3-(3-Methylbutyl)cyclohexan-1-on (2) | 15.00 |
| DAMASCONE DELTA | 2.00 |
| BENZYLACETAT | 15.00 |
| HEDION | 150.00 |
| VELOUTONE | 1.50 |
| HEXYLSALICYLAT | 15.00 |
| UNDECAVERTOL | 2.50 |
| ISOEUGENOLACETAT | 0.50 |
| AGRUMEX LC | 25.00 |
| ORYCLON SPEZIAL | 10.00 |
| HERBYLPROPIONAT | 2.00 |
| SANDRANOL^{®} | 25.00 |
| ETHYLENBRASSYLAT | 25.00 |
| GLOBALIDE^{®} | 25.00 |
| DIPROPYLENGLYCOL | 389.50 |

### Beispiel 19: Parfum 7

| | |
|---|---|
| IONONE BETA REPL BY DPG | 5.00 |
| ESSIGSAEURE | 1.00 |
| ALDEHYD C10 | 1.00 |
| ALDEHYD C11 UNDECANAL | 0.50 |
| ALDEHYD C12 LAURIN | 3.00 |
| HEXENOL CIS-3 | 5.00 |
| HEXENYLACETAT CIS-3 | 5.00 |
| VERTOCITRAL | 5.00 |
| VERTACETAL | 0.50 |
| STYROLYLACETAT | 15.00 |
| STYROLYLPROPIONAT | 0.50 |
| ISOPROPYLMETHYLTHIAZOL-2,4 10% DPG | 1.00 |
| MELONAL 10% DPG | 2.00 |
| DIHYDROMYRCENOL | 25.00 |
| CITRONENOEL ITAL. | 5.00 |
| ORANGENOEL | 5.00 |
| AMAROCIT^{®} | 25.00 |
| CITRORANGE BASE COLIPA | 5.00 |
| MENTHOL L DEST. | 1.00 |
| ETHYLACETAT | 2.00 |
| HEXYLACETAT | 5.00 |
| ISOAMYLACETAT 100% | 1.00 |
| ISOBUTYLACETAT 10% DPG | 2.00 |
| JASMAPRUNAT | 2.50 |
| PRENYLACETAT | 0.50 |
| BUTYLBUTYRAT | 2.00 |
| HEXYLBUTYRAT | 3.00 |
| CAPRONSAEURE NAT. | 1.00 |
| ALDEHYD C14 SOG | 15.00 |
| DECALACTON GAMMA | 2.00 |
| ETHYLMETHYLBUTYRAT-2 | 1.50 |
| SYMFRESH^{®} NX | 25.00 |
| ALLYLCAPRONAT | 5.00 |
| ALLYLCYCLOHEXYLPROPIONAT | 8.00 |
| ALLYLHEPTYLAT | 4.00 |
| THIOMENTHANON-8,3 1% TEC | 2.00 |
| ETHYLMALTOL | 5.00 |
| PASSIONFRUIT TYPE BASE | 25.00 |
| MAJANTOL^{®} | 15.00 |
| TETRAHYDROLINALOOL | 50.00 |
| DIMETHYLBENZYLCARBINYLACETAT | 10.00 |
| 3-(3-Methylbutyl)cyclohexan-1-ol (3) | 1.00 |
| (4-ISOPENTYLCYCLOHEXYL)METHANOL | 15.00 |
| DAMASCONE DELTA | 2.00 |
| BENZYLACETAT | 15.00 |
| HEDION | 150.00 |
| VELOUTONE | 1.50 |
| HEXYLSALICYLAT | 15.00 |
| UNDECAVERTOL | 2.50 |
| ISOEUGENOLACETAT | 0.50 |
| AGRUMEX LC | 25.00 |
| ORYCLON SPEZIAL | 10.00 |
| HERBYLPROPIONAT | 2.00 |
| SANDRANOL^{®} | 25.00 |
| ETHYLENBRASSYLAT | 25.00 |
| GLOBALIDE^{®} | 25.00 |
| DIPROPYLENGLYCOL | 389.50 |

### Beispiel 20: Parfum 8

| | |
|---|---|
| IONONE BETA 50 % DPG | 25.00 |
| FARENAL^{®} 10% DPG | 10.00 |
| FLORAZON | 1.00 |
| HEXENOL CIS-3 | 1.00 |
| HEXENYLACETAT CIS-3 | 1.00 |
| LIGUSTRAL | 5.00 |
| ALLYLAMYLGLYCOLAT | 6.00 |
| CYCLOGALBANAT^{®} | 6.00 |
| MELONAL 10% DPG | 6.00 |
| DIHYDROMYRCENOL | 32.00 |
| LINALYLACETAT | 10.00 |
| OXANTHIA 50% IN TEC 10% DPG | 3.00 |
| HEXYLACETAT | 25.00 |
| ISOAMYLACETAT | 2.00 |
| ETHYLBUTYRAT | 2.00 |
| ETHYLCAPRONAT | 2.00 |
| ALDEHYD C14 SOG | 15.00 |
| ALDEHYD C18 SOG. | 1.00 |
| DECALACTON GAMMA | 6.00 |
| APPLE RED AROMABASE | 4.00 |
| ETHYLMETHYLBUTYRAT-2 | 5.00 |
| MANZANATE | 5.00 |
| ALLYLCYCLOHEXYLPROPIONAT | 5.00 |
| ALLYLHEPTYLAT | 7.00 |
| PRUNELLA TYPE BASE | 12.00 |
| FRAMBINON^{®} | 10.00 |
| ETHYLMALTOL 1% DPG | 10.00 |
| CYCLAMENALDEHYD | 5.00 |
| CALONE 10% DPG | 6.00 |
| HELIONAL | 45.00 |
| FLORHYDRAL | 2.50 |
| FLOROSA | 25.00 |
| ETHYLLINALOOL | 50.00 |
| DIMETHYLBENZYLCARBINYLACETAT | 8.00 |
| TERPINEOL REIN | 12.00 |
| 3-(3-Methylbutyl)cyclohexan-1-ol (3) | 1.00 |
| PHENYLETHYLALKOHOL | 56.00 |
| PHENOXANOL | 15.00 |
| (1-ISOPENTYLCYCLOHEXYL)METHANOL | 1.00 |
| DAMASCON ALPHA | 1.00 |
| DAMASCONE DELTA | 1.00 |
| BENZYLACETAT | 10.00 |
| HEDION | 140.00 |
| AMYLSALICYLAT | 65.00 |
| UNDECAVERTOL | 2.50 |
| IONON ALPHA | 10.00 |
| ISOEUGENOLACETAT | 6.00 |
| AGRUMEX LC | 30.00 |
| SANDRANOL^{®} | 6.00 |
| EVERNYL 10% DPG | 6.00 |
| AMBROX DL 10% DPG | 3.00 |
| AMBRETTOLIDE | 7.00 |
| ETHYLENBRASSYLAT | 95.00 |
| GLOBALIDE^{®} | 50.00 |
| INDOFLOR^{®} KRIST. 10% DPG | 5.00 |
| DIPROPYLENGLYCOL | 119.00 |

Alle Parfum-Beispiele zeigen fruchtige Geruchsmischungen. Die Parfum-Beispiele 5 bis 8 zeichnen sich durch besonders verstärkte Fruchtnoten aus.

## Patentansprüche

1. Verwendung einer Verbindung der Formel (I), 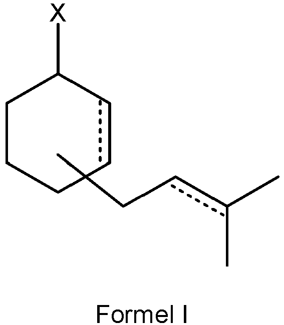 wobei
X eine -CHO, eine -OH, oder eine -CH₂OH Gruppe darstellt, am jeweiligen Ort einer der gestrichelt dargestellten Linien jeweils eine Einfachbindung oder eine Doppelbindung vorliegt, wobei wenn X = OH Gruppe, mindestens eine Doppelbindung vorliegt, und wobei der 3-Isopentyl- oder 3-Isopent-2-enyl-Rest in ortho-, meta-, oder para-Stellung zur X-Gruppe mit dem Ring verbunden ist, als Riech- und/oder Aromastoff.

2. Verwendung einer Verbindung der Formel (I) gemäß Anspruch 1, wobei X eine -CHO, eine -OH, oder eine -CH₂OH Gruppe darstellt, und am jeweiligen Ort einer der gestrichelt dargestellten Linien mindestens eine Doppelbindung vorliegt, als Riech- und/oder Aromastoff.

3. Verwendung einer Verbindung der Formel (I) gemäß Anspruch 1, wobei X eine -CHO oder eine -CH₂OH Gruppe darstellt, und am jeweiligen Ort einer der gestrichelt dargestellten Linien jeweils eine Einfachbindung oder eine Doppelbindung vorliegt, als Riech- und/oder Aromastoff.

4. Verwendung einer Verbindung der Formel (I) gemäß Anspruch 1 bis 3, als Rosen-Noten Riechstoff.

5. Verwendung einer Verbindung der Formel (I) gemäß Anspruch 1 bis 3, zum Vermitteln, Modifizieren und/oder Verstärken einer, zweier oder mehrerer Geruchsnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig.

6. Riech- und/oder Aromastoff umfassend mindestens eine Verbindung der Formel (I) wie in einem der Ansprüche 1 bis 3 definiert.

7. Riech- und/oder Aromastoffmischung mit mindestens einem Riech- und/oder Aromastoff nach Anspruch 6, weiterhin umfassend einen oder mehrere zusätzliche Riech- und/oder Aromastoffe, wobei die zusätzlichen bzw. ein, mehrere oder sämtliche Riechstoffe und/oder Aromastoffe ausgewählt ist bzw. sind aus der Gruppe bestehend aus: Extrakten aus natürlichen Rohstoffen, vorzugsweise Etherische Öle, Concretes, Absolues, Resine, Resinoide, Balsame, Tinkturen und/oder Einzel-Riechstoffen, vorzugsweise aus der Gruppe (1), Kohlenwasserstoffen; (2) Aliphatischen Alkoholen; (3) Aliphatischen Aldehyden und deren Acetalen; (4) Aliphatischen Ketonen und deren Oximen; (5) Aliphatischen schwefelhaltigen Verbindungen; (6) Aliphatischen Nitrilen; (7) Ester von aliphatischen Carbonsäuren; (8) Acyclischen Terpenalkoholen; (9) Acyclischen Terpenaldehyden und -ketonen; (10) Cyclischen Terpenalkoholen; (11) Cyclischen Terpenaldehyden und -ketonen; (12) Cyclischen Alkoholen; (13) Cycloaliphatischen Alkoholen; (14) Cyclischen und cycloaliphatischen Ethern; (15) Cyclischen und makrocyclischen Ketonen; (16) Cycloaliphatischen Aldehyden; (17) Cycloaliphatischen Ketonen; (18) Ester cyclischer Alkohole; (19) Ester cycloaliphatischer Alkohole; (20) Ester cycloaliphatischer Carbonsäuren; (21) Araliphatischen Alkoholen; (22) Ester von araliphatischen Alkoholen und aliphatischen Carbonsäuren; (23) Araliphatische Ether; (24) Aromatischen und araliphatischen Aldehyden; (25) Aromatischen und araliphatischen Ketonen; (26) Aromatischen und araliphatischen Carbonsäuren und deren Ester; (27) Stickstoffhaltigen aromatischen Verbindungen; (28) Phenolen, Phenylethern und Phenylestern; (29) Heterocyclischen Verbindungen; (30) Lactone; sowie deren Gemischen.

8. Riech- und/oder Aromastoffkomposition umfassend:
(a) eine oder mehrere Verbindungen der Formel (I) nach Anspruch 1 bis 3 und/oder 2-(3-Methylbutyl)cyclohexan-1-ol und/oder 3-(3-Methylbutyl)cyclohexan-1-ol und/oder 4-(3-Methylbutyl)cyclohexan-1-ol sowie
(b) einen oder mehrere, vorzugsweise zwei, drei, vier, fünf, sechs, sieben, acht, neun, zehn oder mehr, weitere Riech- und/oder Aromastoffe.

9. Riech- und/oder Aromastoffkomposition nach Anspruch 8, wobei die eine oder mehrere weitere Riech- und/oder Aromastoffe ausgewählt ist bzw. sind aus der Gruppe bestehend aus: Ethyl 2-cyclopent-2-en-1-ylacetat, 1-Cyclohexylethyl (E)-but-2-enoat, (2-Cyclopentylcyclopentyl) (E)-But-2-enoat, Allyl 3-cyclohexylpropanoat, Allylhexanoat, 1,3-Dimethylbutyl (E)-but-2-enoat, 1,3-Dimethylbut-3-enyl 2-methylpropanoat, und/oder (E)-1-(2,6,6-Trimethylcyclohex-2-en-1-yl)but-2-en-1-on.

10. Parfümierter oder aromatisierter Artikel, umfassend:
- eine oder mehrere Verbindungen der Formel (I) nach Anspruch 1 bis 3 und/oder 2-(3-Methylbutyl)cyclohexan-1-ol und/oder 3-(3-Methylbutyl)cyclohexan-1-ol und/oder 4-(3-Methylbutyl)cyclohexan-1-ol und/oder eine Riech- und/oder Aromastoffmischung nach Anspruch 7 und/oder Riech- und/oder Aromastoffkomposition nach Anspruch 8 oder 9 in einer sensorisch wirksamen Menge,
- einen, zwei, drei, vier, fünf, sechs, sieben, acht, neun, zehn oder mehr weitere Riech- und/oder Aromastoffe, wobei vorzugsweise einer, mehrere oder sämtliche der weiteren Riech- und/oder Aromastoffe einen fruchtigen und/oder holzigen Geruch vermitteln,
- einen oder mehrere weitere Zusatz-, Hilfs- und/oder Wirkstoffe, vorzugsweise zwei, drei, vier, fünf oder mehr Zusatz-, Hilfs- und/oder Wirkstoffe.

11. Parfümierter oder aromatisierter Artikel, gemäß Anspruch 10, wobei der parfümierte oder aromatisierte Artikel ausgewählt ist aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

12. Parfümierter oder aromatisierter Artikel nach Anspruch 10 oder 11, wobei die Gesamtmenge an Verbindungen der Formel (I) und/oder 2-(3-Methylbutyl)cyclohexan-1-ol und/oder 3-(3-Methylbutyl)cyclohexan-1-ol und/oder 4-(3-Methylbutyl)cyclohexan-1-ol im Bereich von 0,00001 bis 10 Gew.-% liegt, vorzugsweise im Bereich von 0.0001 bis 5 Gew.-%, besonders bevorzugt im Bereich von 0.001 bis 2 Gew.-%, am meisten bevorzugt 0.005 bis 1 Gew.-%, jeweils bezogen auf die Gesamtmasse des Artikels.

13. Verbindungen der Formel (II) 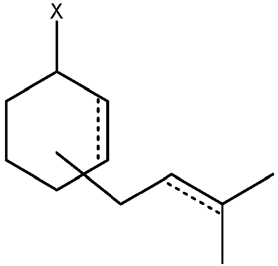 wobei, X eine -CHO oder eine -CH₂OH -Gruppe darstellt, und am Ort einer der gestrichelt dargestellten Linien jeweils eine Einfachbindung oder eine Doppelbindung vorliegt, und wobei der 3-Isopentyl- oder 3-Isopent-2-enyl-Rest in ortho-, meta-, oder para-Stellung zur X-Gruppe mit dem Ring verbunden ist unter der Voraussetzung, dass wenn eine Doppelbindung im Ring vorhanden ist, dann der 3-Isopentyl- oder 3-Isopent-2-enyl-Rest als ortho- oder meta-Substituent direkt an dieser Doppelbindung gebunden ist.

14. Verbindungen der Formel (I) nach Anspruch 1 bis 3 oder der Formel (II) nach Anspruch 13, wobei die Verbindungen in Form eines cis:trans-Isomerengemisches an der 2-, 3-, oder 4-Position vorliegen, mit überwiegender trans-Konfiguration.

15. Verfahren zur Herstellung einer Verbindung der Formel (II) nach Anspruch 13 oder 14, umfassend zumindest die Schritte (i), (ii), **dadurch gekennzeichnet, dass** ausgehend von 2-Cyclohex-3-en durch:
(i) Wittig-Reaktion 1-Isopentyl-2-(methoxymethylen)cyclohexan (Verbindung 4a), 1-Isopentyl-3-(methoxymethylen)cyclohexan (Verbindung 4b), oder 1-Isopentyl-4-(methoxymethylen)cyclohexan (Verbindung 4c) erhalten wird,
(ii) anschließende Enolspaltung zum Aldehyd (Verbindung 5a, b, c) führt; und
(iii) durch optional daran anschließende Reduktion der Alkohol (Verbindung 6a, b, c) erhalten wird.

## Claims

1. Use of a compound of Formula (1), 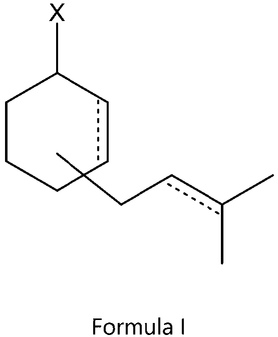 wherein X represents a -CHO, -OH, or -CH₂OH group, a single bond or a double bond is present at the location of each of the dashed lines, wherein if X = OH group, at least one double bond is present, and wherein the 3-isopentyl or 3-isopent-2-enyl residue is connected to the ring in the ortho, meta, or para position to the X group, as a fragrance and/or flavoring agent.

2. Use of a compound of Formula (I) according to claim 1, wherein X represents a CHO, - OH, or -CH₂OH group, and at least one double bond is present at the location of each of the dashed lines, as a fragrance and/or flavoring agent.

3. Use of a compound of Formula (I) according to claim 1, wherein X represents a CHO or a -CH₂OH group, and a single bond or a double bond is present at the location of each of the dashed lines, as a fragrance and/or flavoring agent.

4. Use of a compound of Formula (I) according to claims 1 to 3 as a rose-note fragrance.

5. Use of a compound of Formula (I) according to claims 1 to 3 for conveying, modifying, and/or enhancing one, two, or more odor notes selected from the group consisting of green, herbaceous, fresh, fruity, floral, woody, sweet, earthy, oily, metallic, and balsamic notes, preferably at least one of the notes being green and/or fruity.

6. A fragrance and/or flavoring agent comprising at least one compound of Formula (I) as defined in any one of claims 1 to 3.

7. Fragrance and/or flavoring mixture comprising at least one fragrance and/or flavoring agent according to claim 6, further comprising one or more additional fragrance and/or flavoring agents, wherein the additional or one, several or all of the fragrance and/or flavoring agents is/are selected from the group consisting of: extracts from natural raw materials, preferably essential oils, concretes, absolutes, resins, resinoids, balsams, tinctures and/or individual fragrances, preferably from the group consisting of: (1) hydrocarbons; (2) aliphatic alcohols; (3) aliphatic aldehydes and their acetals; (4) aliphatic ketones and their oximes; (5) aliphatic sulfur-containing compounds; (6) aliphatic nitriles; (7) esters of aliphatic carboxylic acids; (8) acyclic terpene alcohols; (9) acyclic terpene aldehydes and ketones; (10) cyclic terpene alcohols; (11) Cyclic terpene aldehydes and ketones; (12) Cyclic alcohols; (13) Cycloaliphatic alcohols; (14) Cyclic and cycloaliphatic ethers; (15) Cyclic and macrocyclic ketones; (16) Cycloaliphatic aldehydes; (17) Cycloaliphatic ketones; (18) Esters of cyclic alcohols; (19) Esters of cycloaliphatic alcohols; (20) Esters of cycloaliphatic carboxylic acids; (21) Araliphatic alcohols; (22) Esters of araliphatic alcohols and aliphatic carboxylic acids; (23) Araliphatic ethers; (24) Aromatic and araliphatic aldehydes; (25) Aromatic and araliphatic ketones; (26) Aromatic and araliphatic carboxylic acids and their esters; (27) Nitrogen-containing aromatic compounds; (28) Phenols, phenyl ethers, and phenyl esters; (29) heterocyclic compounds; (30) lactones; and mixtures thereof.

8. Fragrance and/or flavoring composition comprising:
(a) one or more compounds of Formula (I) according to claims 1 to 3 and/or 2-(3-methylbutyl)cyclohexan-1-ol and/or 3-(3-methylbutyl)cyclohexan-1-ol and/or 4-(3-methylbutyl)cyclohexan-1-ol, and
(b) one or more, preferably two, three, four, five, six, seven, eight, nine, ten, or more, further fragrance and/or flavoring agents.

9. Fragrance and/or flavoring composition according to claim 8, wherein one or more further fragrance and/or flavoring agents are selected from the group consisting of: ethyl 2-cyclopent-2-en-1-yl acetate, 1-cyclohexylethyl (E)-but-2-enoate, (2-cyclopentylcyclopentyl) (E)-but-2-enoate, allyl 3-cyclohexyl propanoate, allyl hexanoate, 1,3-dimethylbutyl (E)-but-2-enoate, 1,3-dimethylbut-3-enyl 2-methyl propanoate, and/or (E)-1-(2,6,6-trimethylcyclohex-2-en-1-yl)but-2-en-1-one.

10. Perfumed or flavored article comprising:
- one or more compounds of Formula (I) according to claims 1 to 3 and/or 2-(3-methylbutyl)cyclohexan-1-ol and/or 3-(3-methylbutyl)cyclohexan-1-ol and/or 4-(3-methylbutyl)cyclohexan-1-ol and/or a fragrance and/or flavoring mixture according to claim 7 and/or a fragrance and/or flavoring composition according to claim 8 or 9 in a sensorially effective amount,
- one, two, three, four, five, six, seven, eight, nine, ten or more further fragrance and/or flavoring substances, wherein preferably one, several or all of the further fragrance and/or flavoring substances impart a fruity and/or woody odor,
- one or more further additives, excipients and/or active ingredients, preferably two, three, four, five or more additives, excipients and/or active ingredients.

11. Perfumed or flavored article according to claim 10, wherein the perfumed or flavored article is selected from the group consisting of:
perfume extracts, eau de parfums, eau de toilettes, aftershaves, eau de colognes, pre-shave products, splash colognes, perfumed refreshing wipes, acidic, alkaline or neutral detergents, fabric refreshers, ironing aids, liquid detergents, powdered detergents, laundry pre-treatments, fabric softeners, washing soaps, laundry tablets, disinfectants, surface disinfectants, air fresheners, aerosol sprays, waxes and polishes, body care products, hand creams and lotions, foot creams and lotions, depilatory creams and lotions, aftershave creams and lotions, tanning creams and lotions, hair care products, deodorants, antiperspirants, decorative cosmetic products, candles, Lamp oils, incense sticks, insecticides, repellents, and fuels.

12. Perfumed or flavored article according to claim 10 or 11, wherein the total amount of compounds of Formula (I) and/or 2-(3-methylbutyl)cyclohexan-1-ol and/or 3-(3-methylbutyl)cyclohexan-1-ol and/or 4-(3-methylbutyl)cyclohexan-1-ol is in the range of 0.00001 to 10 wt.%, preferably in the range of 0.0001 to 5 wt.%, particularly preferably in the range of 0.001 to 2 wt.%, most preferably 0.005 to 1 wt.%, in each case based on the total mass of the article.

13. Compounds of Formula (II) 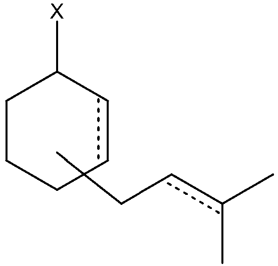 wherein X represents a -CHO or a -CH₂OH group; and a single bond or a double bond is present at the location of each of the dashed lines, wherein the 3-isopentyl or 3-isopent-2-enyl residue is connected to the ring in the ortho, meta, or para position to the X group, provided that if a double bond is present in the ring, then the 3-isopentyl or 3-isopent-2-enyl residue is directly bonded to this double bond as an ortho or meta substituent.

14. Compounds of Formula (I) according to claims 1 to 3 or of Formula (II) according to claim 13, wherein the compounds are present as a cis:trans isomer mixture at the 2-, 3-, or 4-position, with a predominant trans configuration.

15. A process for the preparation of a compound of Formula (II) according to claim 13 or 14, comprising at least steps (i), (ii), **characterized in that**, starting from 2-cyclohex-3-ene,
(i) 1-isopentyl-2-(methoxymethylene)cyclohexane (compound 4a), 1-isopentyl-3-(methoxymethylene)cyclohexane (compound 4b), or 1-isopentyl-4-(methoxymethylene)cyclohexane (compound 4c) is obtained by Wittig-reaction.
(ii) subsequent enol cleavage leads to the aldehyde (compound 5a, b, c); and
(iii) the alcohol (compound 6a, b, c) is obtained by optional subsequent reduction.

## Revendications

1. Utilisation d'un composé de formule (I), 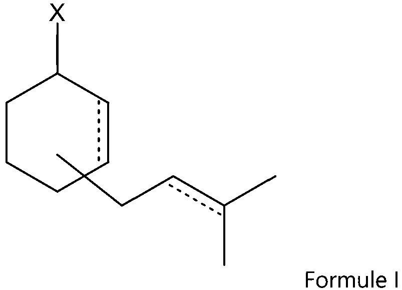 dans laquelle
X représente un groupe -CHO, -OH ou -CH₂OH, il existe une liaison simple ou une liaison double à l'emplacement respectif de chacune des lignes représentées en pointillés, dans lequel, lorsque X = groupe OH, il existe au moins une liaison double, et dans lequel le radical 3-isopentyle ou 3-isopent-2 -ényle est lié au cycle en position ortho, méta ou para par rapport au groupe X, comme substance odorante et/ou aromatique.

2. Utilisation d'un composé de formule (I) selon la revendication 1, dans lequel X représente un groupe -CHO, -OH ou -CH₂OH, et au moins une double liaison est présente à l'emplacement respectif de l'une des lignes représentées en pointillés, comme substance odorante et/ou aromatique.

3. Utilisation d'un composé de formule (I) selon la revendication 1, dans lequel X représente un groupe -CHO ou -CH₂OH, et à l'emplacement respectif de l'une des lignes représentées en pointillés, il existe respectivement une liaison simple ou une double liaison, comme substance odorante et/ou aromatique.

4. Utilisation d'un composé de formule (I) selon les revendications 1 à 3, comme substance odorante à notes de rose.

5. Utilisation d'un composé de formule (I) selon les revendications 1 à 3, pour conférer, modifier et/ou renforcer une, deux ou plusieurs notes olfactives choisies dans le groupe constitué par les notes vertes, herbacées, fraîches, fruitées, florales, boisées, sucrées, terreuses, grasses, métalliques et balsamiques, de préférence au moins l'une des notes vertes et/ou fruitées.

6. Substance odorante et/ou aromatique comprenant au moins un composé de formule (I) tel que défini dans l'une des revendications 1 à 3.

7. Mélange de substances odorantes et/ou aromatiques comprenant au moins une substance odorante et/ou aromatique selon la revendication 6, comprenant en outre une ou plusieurs substances odorantes et/ou aromatiques supplémentaires, les substances odorantes et/ou aromatiques supplémentaires, respectivement une, plusieurs ou toutes, étant choisies dans le groupe constitué par : des extraits de matières premières naturelles, de préférence des huiles essentielles, des concrètes, des absolues, des résines, des résinoïdes, des baumes, des teintures et/ou des substances odorantes individuelles, de préférence du groupe (1), des hydrocarbures ; (2) des alcools aliphatiques ; (3) des aldéhydes aliphatiques et leurs acétals ; (4) cétones aliphatiques et leurs oximes ; (5) composés aliphatiques soufrés ; (6) nitriles aliphatiques ; (7) esters d'acides carboxyliques aliphatiques ; (8) alcools terpéniques acycliques ; (9) aldéhydes et cétones terpéniques acycliques ; (10) alcools terpéniques cycliques ; (11) aldéhydes et cétones terpéniques cycliques ; (12) alcools cycliques ; (13) alcools cycloaliphatiques ; (14) éthers cycliques et cycloaliphatiques ; (15) cétones cycliques et macrocycliques ; (16) aldéhydes cycloaliphatiques ; (17) cétones cycloaliphatiques ; (18) esters d'alcools cycliques ; (19) esters d'alcools cycloaliphatiques ; (20) esters d'acides carboxyliques cycloaliphatiques ; (21) alcools araliphatiques ; (22) Esters d'alcools araliphatiques et d'acides carboxyliques aliphatiques ; (23) Éthers araliphatiques ; (24) Aldéhydes aromatiques et araliphatiques ; (25) Cétones aromatiques et araliphatiques ; (26) Acides carboxyliques aromatiques et araliphatiques et leurs esters ; (27) Composés aromatiques azotés ; (28) Phénols, éthers phényliques et esters phényliques ; (29) Composés hétérocycliques ; (30) Lactones ; ainsi que leurs mélanges.

8. Composition odorante et/ou aromatique comprenant :
(a) un ou plusieurs composés de formule (I) selon les revendications 1 à 3 et/ou du 2-(3-méthylbutyl)cyclohexane-1-ol et/ou du 3-(3-méthylbutyl)cyclohexane-1-ol et/ou du 4-(3-méthylbutyl)cyclohexane-1-ol, ainsi que
(b) un ou plusieurs, de préférence deux, trois, quatre, cinq, six, sept, huit, neuf, dix ou plus, autres substances odorantes et/ou aromatiques.

9. Composition odorante et/ou aromatique selon la revendication 8, dans laquelle la ou les autres substances odorantes et/ou aromatiques sont choisies dans le groupe constitué par : l'acétate d'éthyle 2-cyclopent-2-én-1-yle, le (E)-but-2-énoate de 1-cyclohexylethyle, le (E)-but-2-énoate de (2-cyclopentylcyclopentyle), le propanoate d'allyle 3-cyclohexyle, hexanoate d'allyle, 1,3-diméthylbutyl (E)-but-2-énoate, 1,3-diméthylbut-3-ényl 2-méthylpropanoate, et/ou (E)-1-(2,6,6-triméthylcyclohex-2-én-1-yl)but-2-én-1-one.

10. Article parfumé ou aromatisé comprenant :
- un ou plusieurs composés de formule (I) selon les revendications 1 à 3 et/ou 2-(3-méthylbutyl)cyclohexane-1-ol et/ou 3-(3-méthylbutyl)cyclohexane -1-ol et/ou du 4-(3-méthylbutyl)cyclohexane-1-ol et/ou un mélange de substances odorantes et/ou aromatiques selon la revendication 7 et/ou une composition de substances odorantes et/ou aromatiques selon la revendication 8 ou 9 en une quantité efficace sur le plan sensoriel,
- une, deux, trois, quatre, cinq, six, sept, huit, neuf, dix ou plusieurs autres substances odorantes et/ou aromatiques, une, plusieurs ou toutes les autres substances odorantes et/ou aromatiques conférant de préférence une odeur fruitée et/ou boisée,
- un ou plusieurs autres additifs, excipients et/ou principes actifs, de préférence deux, trois, quatre, cinq ou plus d'additifs, excipients et/ou principes actifs.

11. Article parfumé ou aromatisé selon la revendication 10, l'article parfumé ou aromatisé étant choisi dans le groupe constitué par :
des extraits de parfum, des eaux de parfum, des eaux de toilette, des lotions après-rasage, des eaux de Cologne, des produits avant-rasage, des splash Cologne, des lingettes rafraîchissantes parfumées, des détergents acides, alcalins ou neutres, des rafraîchisseurs textiles, des aides au repassage, des lessives liquides, des lessives en poudre, produits de prétraitement du linge, adoucissants, savons de lessive, tablettes de lessive, désinfectants, désinfectants de surface, désodorisants, sprays aérosols, cires et polish, produits de soins corporels, crèmes et lotions pour les mains, crèmes et lotions pour les pieds, crèmes et lotions dépilatoires, crèmes et lotions après-rasage, crèmes et lotions bronzantes, produits de soins capillaires, déodorants, antisudorifiques, produits cosmétiques décoratifs, bougies, huiles pour lampes, bâtons d'encens, insecticides, répulsifs et carburants.

12. Article parfumé ou aromatisé selon la revendication 10 ou 11, dans lequel la quantité totale de composés de formule (I) et/ou de 2-(3-méthylbutyl)cyclohexane-1-ol et/ou de 3-(3-méthylbutyl)cyclohexane-1-ol et/ou de 4-(3-méthylbutyl)cyclohexane -1-ol est comprise entre 0,00001 et 10 % en poids, de préférence entre 0,0001 et 5 % en poids, de manière particulièrement préférée entre 0,001 et 2 % en poids, et de manière encore plus préférée entre 0,005 et 1 % en poids, dans chaque cas par rapport à la masse totale de l'article.

13. Composés de formule (II) 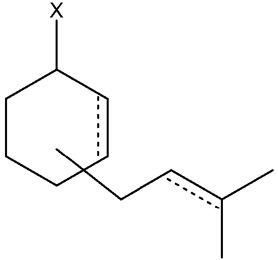 dans laquelle X représente un groupe -CHO ou -CH₂OH ; et à l'emplacement de chacune des lignes représentées en pointillés, il y a une liaison simple ou une liaison double, et dans laquelle le radical 3-isopentyle ou 3-isopent-2 -ényle est lié au cycle en position ortho, méta ou para par rapport au groupe X, à condition que, lorsqu'une double liaison est présente dans le cycle, le radical 3-isopentyle ou 3-isopent-2-ényle soit lié directement à cette double liaison en tant que substituant ortho ou méta.

14. Composés de formule (I) selon les revendications 1 à 3 ou de formule (II) selon la revendication 13, les composés se présentant sous la forme d'un mélange d'isomères cis:trans en position 2, 3 ou 4, avec une configuration trans prédominante.

15. Procédé de préparation d'un composé de formule (II) selon la revendication 13 ou 14, comprenant au moins les étapes (i), (ii), **caractérisé en ce que**, à partir du 2-cyclohex-3-ène, par:
(i) réaction de Wittig du 1-isopentyl-2-(méthoxyméthylène)cyclohexane (composé 4a), 1-isopentyl-3-(méthoxyméthylène)cyclohexane (composé 4b), ou 1-isopentyl-4-(méthoxyméthylène)cyclohexane (composé 4c) est obtenu,
(ii) suivie d'une scission de l'énol en aldéhyde (composé 5a, b, c) ; et
(iii) suivie éventuellement d'une réduction pour obtenir l'alcool (composé 6a, b, c).
